(19) Europäisches Patentamt
European Patent Office
Office européen des brevets

(11) Publication number: **0 114 027**
**B1**

(12) **EUROPEAN PATENT SPECIFICATION**

(45) Date of publication of patent specification: **07.01.88**

(51) Int. Cl.$^4$: **C 07 D 209/18,**
C 07 D 405/04, A 61 K 31/405

(21) Application number: **83810548.4**

(22) Date of filing: **22.11.83**

(54) **Analogs of mevalolactone and derivatives thereof, processes for their production, pharmaceutical compositions containing them and their use as pharmaceuticals.**

(30) Priority: **22.11.82 US 443668**
**04.11.83 US 548850**

(43) Date of publication of application:
**25.07.84 Bulletin 84/30**

(45) Publication of the grant of the patent:
**07.01.88 Bulletin 88/01**

(84) Designated Contracting States:
**AT BE CH DE FR GB IT LI LU NL SE**

(56) References cited:

**TETRAHEDRON LETTERS, vol. 23, no. 42, 1982, Pergamon Press, Oxford, GB YUH-LIN YANG et al.: "Mevinic acids and analogues: preparation of a key chiral intermediate", p. 4305, 4308**

(73) Proprietor: **SANDOZ AG**
**Lichtstrasse 35**
**CH-4002 Basel (CH)**
(84) **BE CH FR GB IT LI LU NL SE**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN Verwaltungsgesellschaft m.b.H.**
**Brunner Strasse 59**
**A-1235 Wien (AT)**
(84) **AT**

(72) Inventor: **Kathawala, Faizulla Gulamhusein**
**39 Woodland Avenue**
**Mountain Lakes, N.J., 07946 (US)**

**EP 0 114 027 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention concerns heterocyclic analogs of mevalono-lactone and derivatives thereof, process for their production, pharmaceutical compositions containing them and their use as pharmaceuticals, in particular as hypolipoproteinemic and antiatherosclerotic agents.

The invention is especially concered with compounds of formula I

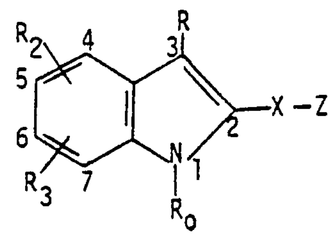

(I)

wherein one of R and $R_o$ is

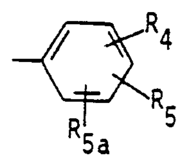

and the other is primary or secondary $C_{1-6}$alkyl, $C_{3-6}$cycloalkyl or phenyl-$(CH_2)_m$-, wherein

$R_4$ is hydrogen, $C_{1-4}$alkyl, $C_{1-4}$alkoxy, (except t-butoxy), trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen, $C_{1-2}$alkyl, $C_{1-2}$alkoxy, fluoro or chloro, and

m is 1, 2 or 3, with the provisos that both $R_5$ and $R_{5a}$ must be hydrogen when $R_4$ is hydrogen, $R_{5a}$ must be hydrogen when $R_5$ is hydrogen, not more than one of $R_4$ and $R_5$ is trifluoromethyl, not more than one of $R_4$ and $R_5$ is phenoxy and not more than one of $R_4$ and $R_5$ is benzyloxy,

$R_2$ is hydrogen $C_{1-4}$alkyl, $C_{3-6}$cycloalkyl, $C_{1-4}$alkoxy, (except t-butoxy), trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that $R_3$ must be hydrogen when $R_2$ is hydrogen, not more than one of $R_2$ and $R_3$ is trifluoromethyl, not more than one of $R_2$ and $R_3$ is phenoxy, and not more than one of $R_2$ and $R_3$ is benzyloxy,

X is $-(CH_2)_n-$ or $-CH{=}CH-$ (n=0, 1, 2 or 3), and

$R_6$
5 4 3 2 1
Z is $-CH-CH_2-C-CH_2-COOH$ II
OH OH

wherein $R_6$ is hydrogen or $C_{1-3}$alkyl in free acid form or in the form of a physiologically-hydrolysable and -acceptable ester of a δ-lactone thereof or in salt form.

By the term "physiologically-hydrolysable and -acceptable ester" is meant an ester of a compound in accordance with the invention in which the carboxyl moiety is esterified, and which is hydrolysable under physiological conditions to yield an alcohol which is itself physiologically acceptable, e.g. non-toxic at desired dosage levels. Preferred such esters as Z can be represented together with the free acid by formula IIa

$R_6$
$-CH-CH_2-C-CH_2-COOR_7$ IIa
OH OH

wherein $R_7$ is hydrogen, $C_{1-4}$alkyl or benzyl, preferably hydrogen, $C_{1-3}$alkyl, n-butyl, i-butyl, t-butyl or benzyl and $R_6$ is as defined above.

When in salt form $R_7$ represents a cation.

When Z is in lactone form it forms a δ-lactone of formula IIb

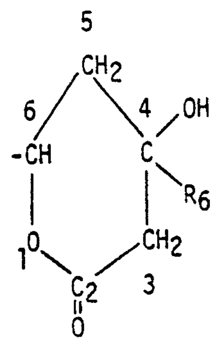

IIb

and references to "lactone" hereinafter refer to δ-lactones.

Salts of the compounds of the invention, e.g. of the compounds of formula I, include in partiuclar their pharmaceutically acceptable salts. Such pharmaceutically acceptable salts include e.g. alkali metal salts such as the sodium and potassium salts and ammonium salts.

References to compounds of formula I and sub-species thereof are intended to cover all forms unless otherwise stated.

The compounds of formula I may be divided into two groups, the compounds of formulae IA and IB:

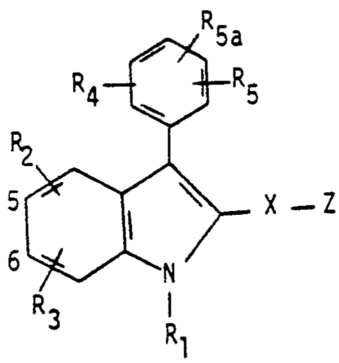

(IA)

and

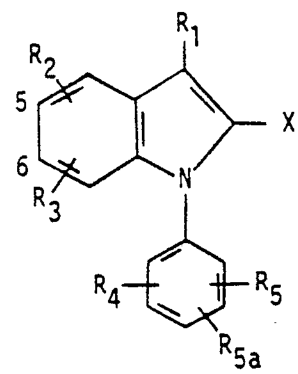

,

(IB)

wherein

$R_1$ is primary or secondary $C_{1-6}$alkyl, $C_{3-6}$cycloalkyl or phenyl-$(CH_2)_m$—, and

$R_2$—$R_{5a}$, X, Z and m are as defined above.

The compounds of formula IA may be divided into a two sub-groups, the compounds wherein Z is a group of formula II in other than lactone form (Group IAa) and those wherein Z is a group of formula (Group IAb) IIb. Likewise, the compounds of formula IB may be divided into two sub-groups, the compounds wherein Z is a group of formula II in other than lactone form (Group IBa) and those wherein Z is a group of formula IIb (Group IBb).

As is self-evident to those in the art, each compound of formula I (and every sub-scope and species thereof) has at least two centers of asymmetry (e.g. the two carbon atoms bearing the hydroxy groups in the group of formula IIa and the carbon atom bearing the hydroxy group and the carbon atom having the free valence in the group of formula IIb) and these lead to four stereoisomeric forms (enantiomers) of each compound (two racemates or pairs of diastereoisomers). These four stereoisomes may be designated as the R, R; R, S; S, R; and S, S enantiomers, all four stereoisomers being within the scope of this invention. Depending on the nature of substituents further asymmetric carbon atoms may be present and the resulting isomers and mixtures thereof also form part of the invention. Compounds containing only two centres of asymmetry (four mentioned stereoisomers) are preferred.

$R_1$ is preferably primary or secondary $C_{1-6}$alkyl not containing an asymmetric carbon atom (e.g. methyl, ethyl, n-propyl, i-propyl, n-butyl, i-butyl, 1-ethylpropyl, neopentyl and n-hexyl), more preferably $C_{1-3}$alkyl and most preferably methyl, ethyl or i-propyl, especially i-propyl.

Alkyl as $R_2$ is preferably $C_{1-3}$ or n-, i- or t-butyl and alkoxy $C_{1-3}$ or n- or i-butoxy. $R_2$ is preferably $R_2'$, where $R_2'$ is hydrogen, $C_{1-3}$alkyl, $C_{1-3}$alkoxy, trifluoromethyl, fluoro, chloro, phenoxy or benzyloxy, more preferably $R_2''$, where $R_2''$ is hydrogen, $C_{1-3}$alkyl, methoxy, fluoro, chloro or 4-, 5- or 6-benzyloxy, and most preferably $R_2'''$, where $R_2'''$ is hydrogen, $C_{1-3}$alkyl or 4- or 6-benzyloxy, especially hydrogen or methyl and most especially hydrogen.

$R_3$ is preferably $R_3'$, where $R_3'$ is hydrogen, $C_{1-3}$alkyl, $C_{1-2}$alkoxy, fluoro or chloro, more preferably $R_3''$,

where $R_3''$ is hydrogen or $C_{1-3}$alkyl and most preferably $R_3'''$, where $R_3'''$ is hydrogen or methyl, especially hydrogen. $R_3$ ($R_3'$, etc.) must be hydrogen when $R_2$ ($R_2'$, etc.) is hydrogen.

Preferably, when $R_2$ ($R_2'$, $R_2''$, etc.) is other than hydrogen and $R_3$ ($R_3'$, $R_3''$, etc.) is hydrogen, $R_2$ ($R_2'$, etc.) is in the 4-, 5- or 6-position.

Preferably, when both $R_2$ ($R_2'$, $R_2''$, etc.) and $R_3$ ($R_3'$, $R_3''$, etc.) are other than hydrogen, at least one of them is in the 5- or 6-position, neither of them is in the 7-position, and not more than one of them is a member of the group consisting of t-butyl, $C_{3-6}$cycloalkyl, trifluoromethyl, phenoxy and benzyloxy; more preferably, they are not *ortho* to each other when neither of them is a member of the group consisting of methyl, methoxy, fluoro and chloro. Most preferably, one is in the 4-position and the other is in the 6-position.

Except where otherwise indicated: (a) Any $C_{1-4}$alkyl or $C_{3-6}$cycloalkyl group as $R_2$, $R_2'$, $R_3$, $R_3'$, etc. is more preferably in the 4- or 6-position. (b) Any $C_{1-4}$alkoxy, fluoro or chloro substituent as $R_2$, $R_2'$, $R_3$, $R_3'$, etc. is more preferably in the 5-position. (c) Any benzyloxy as $R_2$, $R_2'$, $R_3$, $R_3'$, etc. is more preferably in the 4-, 5- or 6-position and most preferably in the 4- or 6-position, especially the 6-position.

Alkyl as $R_4$ is preferably $C_{1-3}$ or n-, i- or t-butyl and alkoxy $C_{1-3}$ or n- or i-butoxy. $R_4$ is preferably $R_4'$, where $R_4'$ is hydrogen, $C_{1-3}$alkyl, $C_{1-3}$alkoxy, trifluoromethyl, fluoro, chloro, phenoxy or benzyloxy, more preferably $R_4''$, where $R_4''$ is hydrogen, methyl, methoxy, fluoro or chloro, and most preferably $R_4'''$, where $R_4'''$ is hydrogen, methyl or fluoro, especially $R_4''''$, where $R_4''''$ is hydrogen, 3- or 4-methyl or 4-fluoro and most especially 4-fluoro.

$R_5$ is preferably $R_5'$, where $R_5'$ is hydrogen, $C_{1-2}$alkyl, $C_{1-2}$alkoxy, fluoro or chloro, more preferably $R_5''$, where $R_5''$ is hydrogen, methyl, methoxy, fluoro or chloro, and most preferably $R_5'''$, where $R_5'''$ is hydrogen or methyl, especially hydrogen. $R_5$ ($R_5'$, $R_5''$, etc.) must be hydrogen when $R_4$ ($R_4'$, $R_4''$, etc.) is hydrogen.

$R_{5a}$ is preferably $R_{5a}'$, where $R_{5a}'$ is hydrogen or methyl, and most preferably hydrogen. $R_{5a}$ ($R_{5a}'$, etc.) must be hydrogen when at least one of $R_4$ ($R_4'$, $R_4''$, etc.) and $R_5$ ($R_5'$, $R_5''$, etc.) is hydrogen.

Preferably, when $R_4$ ($R_4'$, $R_4''$, etc.) is other than hydrogen and $R_5$ ($R_5'$, $R_5''$, etc.) and $R_{5a}$ ($R_{5a}'$, etc.) are both hydrogen, $R_4$ ($R_4'$, etc.) is in a *meta* or *para* position, more preferably the *para* position. The most preferred monosubstituted phenyl group is 4-fluorophenyl.

Preferably, when both $R_4$ ($R_4'$, $R_4''$, etc.) and $R_5$ ($R_5'$, $R_5''$, etc.) are other than hydrogen and $R_{5a}$ ($R_{5a}'$, etc.) is hydrogen, at least one of $R_4$ ($R_4'$, etc.) and $R_5$ ($R_5'$, etc.) is in a *meta* or *para* position (more preferably both are), and not more than one of them is a member of the group consisting of t-butyl, trifluoromethyl, phenoxy and benzyloxy; more preferably, $R_4$ ($R_4'$, etc.) and $R_5$ ($R_5'$, etc.) are not *ortho* to each other when neither of them is a member of the group consisting of methyl, methoxy, fluoro and chloro. The most preferred disubstituted phenyl groups are 3,4- and 3,5-dimethylphenyl and 4-fluoro-3-methylphenyl, especially 3,5-dimethylphenyl and 4-fluoro-3-methylphenyl.

Preferably, when each of $R_4$ ($R_4'$, etc.), $R_5$ ($R_5'$, etc.) and $R_{5a}$ ($R_{5a}'$, etc.) is other than hydrogen, at least two of them (more preferably, all three) are in *meta* or *para* positions, and not more than one of them is a member of the group consisting of t-butyl, trifluoromethyl, phenoxy and benzyloxy; more preferably, no two fo them are *ortho* to each other unless at least one member of the or each pair of substituents that are *ortho* to each other is a member of the group consisting of methyl, methoxy, fluoro and chloro. The most preferred trisubsituted phenyl group is 3,5-dimethyl-4-fluorophenyl.

$R_6$ is preferably $R_6'$, where $R_6'$ is hydrogen or $C_{1-2}$alkyl, more preferably $R_6''$, where $R_6''$ is hydrogen or methyl, and most preferably hydrogen.

$R_7$ is preferably $R_7'$, where $R_7'$ is hydrogen or $C_{1-3}$alkyl, more preferably $R_7''$, where $R_7''$ is hydrogen or $C_{1-2}$alkyl. Such compounds wherein Z is of formula II or IIa are most preferably in salt form. Preferred salt-forming cations are those free from centres of asymmetry, especially e.g. sodium, potassium or ammonium, most preferably sodium.

X is preferably X', where X is $-(CH_2)_m-$ or

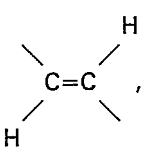 ,

more preferably X'', where X'' is $-CH_2CH_2-$ or

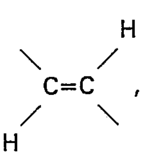 , especially 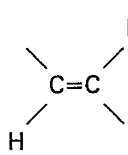 .

Z is preferably a group of formula IIa wherein $R_6$ is $R_6'$ and $R_7$ is $R_7'$ or a group of formula IIb where in $R_6$ is $R_6'$, more preferably a group of formula IIa wherein $R_6$ is $R_6''$ and $R_7$ is $R_7''$ or a group of formula IIb

wherein $R_6$ is $R_6''$ most preferably a group of formula IIa wherein $R_6$ is hydrogen and $R_7$ is $R_7''$ or a group of formula IIb wherein $R_6$ is hydrogen, especially a group of formula IIa wherein $R_6$ is hydrogen in salt form, particularly in sodium salt form, or a group of formula IIb wherein $R_6$ is hydrogen.

n is preferably m, where m is 1, 2 or 3, preferably 2 or 3 and most preferably 2.

Insofar as the compounds of Groups IAa and IBa are concerned, the *erythro* isomers are generally preferred over the *threo* isomers, *erythro* and *threo* referring to the relative positions of the hydroxy groups in the 3- and 5-positions (of the group of formula II or IIa).

As between compounds of formula I having identical R, $R_o$, $R_2$, $R_3$, $R_6$ and X groups, free acid, salt and ester forms are generally preferred to lactone forms.

The preferred stereoisomers of the compounds having only two assymetric carbons wherein X is a direct bond or —CH=CH—, and Z is in other than lactone form are the 3R,5S and 3R,5R isomers and the racemate of which each is a constituent, i.e., the 3R,5S-3S,5R (*erythro*) and 3R,5R-3S,5S (*threo*) racemates, with the 3R,5S isomer and the racemate of which it is a constituent being more preferred and the 3R,5S isomer being most preferred.

The preferred stereoisomers of the compounds having only two assymetric carbons wherein X is $—(CH_2)_m—$, and Z is in other than lactone form are the 3R,5R and 3R,5S isomers and the racemate of which each is a constituent, *i.e.,* the 3R,5R-3S,5S (*erythro*) and 3R,5S-3S,5R (*threo*) racemates, with the 3R,5R isomer and the racemate of which it is a constituent being more preferred and the 3R,5R isomer being most preferred.

The preferred stereoisomers of the compounds having only two assymetric carbons wherein X is a direct bond or —CH=CH—, and Z is a group of formula IIb are the 4R,6S and 4R,6R isomers and the racemate of which each is a constituent, *i.e.*, the 4R,6S-4S,6R (*trans* lactone) and 4R,6R-4S,6S (*cis* lactone) racemates, with the 4R,6S isomer and the racemate of which it is a constituent being more preferred and the 4R,6S isomer being most preferred.

The preferred stereoisomers of the compounds having only two assymetric carbons wherein X is $—(CH_2)_m—$, and Z is a group of formula IIb are the 4R,6R and 4R,6S isomers and the racemate of which each is a constituent, *i.e.,* the 4R,6R-4S,6S (*trans* lactone) and 4R,6S-4S,6R (*cis* lactone) racemates, with the 4R,6R isomer and the racemate of which it is a constituent being more preferred and the 4R,6R isomer being most preferred.

Each of the preferences set forth above applies, not only to the compounds of formula I but also to the compounds of formulae IA and IB and those of Groups IAa, IAb, IBa and IBb as well as to every other subgroup thereof set forth *infra, e.g.,* Groups (i)-(cxiv), unless otherwise indicated. When any preference contains a variable, the preferred significances of that variable apply to the preference in question, unless otherwise indicated.

Preferred groups of compounds of formula I include the compounds

(i) of Group IAa wherein $R_1$ is $R_1'$, $R_2$ is $R_2'$, $R_3$ is $R_3'$, $R_4$ is $R_4'$, $R_5$ is $R_5'$, $R_{5a}$ is $R_{5a}'$, $R_6$ is $R_6'$, $R_7$ is $R_7'$, and X is X'.

(ii) of (i) wherein when $R_2'$ is other than hydrogen and $R_3'$ is hydrogen, $R_2'$ is in the 4-, 5- or 6-position; when both $R_2'$ and $R_3'$ are other than hydrogen, at least one of them is in the 5- or 6-position and neither of them is in the 7-position; when both $R_4'$ and $R_5'$ are other than hydrogen and $R_{5a}'$ is hydrogen, at least one of $R_4'$ and $R_5'$ is in a *meta* or *para* position; and when each of $R_4'$, $R_5'$ and $R_{5a}'$ is other than hydrogen, at least two of them are in *meta* or *para* positions.

(iii)—(iv) of (i) and (ii) wherein $R_6$ is $R_6''$, especially hydrogen,

(v)—(vi) of (i) and (ii) wherein $R_1$ is $C_{1-3}$alkyl, $R_2$ is $R_2''$, $R_3$ is $R_3''$, $R_4$ is $R_4''$, $R_5$ is $R_5''$, $R_6$ is $R_6''$, especially hydrogen, $R_7$ is $R_7''$, and X is X''.

(vii) of (i) wherein $R_1$ is $C_{1-3}$alkyl, $R_2$ is $R_2''$, $R_3$ is $R_3'''$, $R_4$ is $R_4'''$, $R_5$ is $R_5'''$, $R_{5a}$ is hydrogen, $R_6$ is hydrogen, $R_7$ is $R_7''$, and X is

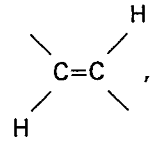 ,

(viii)—(xiii) of (i)—(vi) wherein any salt is a sodium, potassium or ammonium salt,

(xiv) of Group IAb wherein $R_1$ is $R_1'$, $R_2$ is $R_2'$, $R_3$ is $R_3'$, $R_4$ is $R_4'$, $R_5$ is $R_5'$, $R_{5a}$ is $R_{5a}'$, $R_6$ is $R_6'$ and X is X,

(xv) of (xiv) wherein when $R_2'$ is other than hydrogen and $R_3'$ is hydrogen, $R_2'$ is in the 4-, 5- or 6-position; when both $R_2'$ and $R_3'$ are other than hydrogen, at least one of them is in the 5- or 6-position and neither of them is in the 7-position; when both $R_4'$ and $R_5'$ are other than hydrogen and $R_{5a}'$ is hydrogen, at least one of $R_4'$ and $R_5'$ is in a *meta* or *para* position; and when each of $R_4'$, $R_5'$ and $R_{5a}$ is other than hydrogen, at least two of them are in *meta* or *para* positions,

(xvi)—(xvii) of (xiv) and (xv) wherein $R_6$ is $R_6''$, especially hydrogen,

(xviii)—(xix) of (xiv) and (xv) wherein $R_1$ is $C_{1-3}$alkyl, $R_2$ is $R_2''$, $R_3$ is $R_3''$, $R_4$ is $R_4''$, $R_5$ is $R_5''$, $R_6$ is $R_6''$, especially hydrogen, and X is X'',

(xx) of (xiv) wherein $R_1$ is $C_{1-3}$alkyl, $R_2$ is $R_2''$, $R_3$ is $R_3'''$, $R_4$ is $R_4'''$, $R_5$ is $R_5'''$, $R_{5a}$ is hydrogen, $R_6$ is hydrogen, and X is

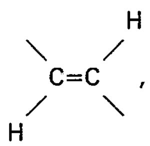

(xxi) of Group IBa wherein $R_1$ is $R_1'$, $R_2$ is $R_2'$, $R_3$ is $R_3'$, $R_4$ is $R_4'$, $R_5$ is $R_5'$, $R_{5a}$ is $R_{5a}'$, $R_6$ is $R_6'$, $R_7$ is $R_7'$, and X is X'.

(xxii) of (xxi) wherein $R_2'$ is other than hydrogen and $R_3'$ is hydrogen, $R_2'$ is in the 4-, 5- or 6-position; when both $R_2'$ and $R_3'$ are other than hydrogen, at least one of them is in the 5- or 6-position and neither of them is in the 7-position; when both $R_4'$ and $R_5'$ are other than hydrogen and $R_{5a}'$ is hydrogen, at least one of $R_4'$ and $R_5'$ is in a *meta* or *para* position; and when each of $R_4'$, $R_5'$ and $R_{5a}'$ is other than hydrogen, at least two of them are in *meta* or *para* positions,

(xxiii)—(xxiv) of (xxi) and (xxii) wherein $R_6$ is $R_6''$, especially hydrogen,

(xxv)—(xxvi) of (xxi) and (xxii) wherein $R_1$ is $C_{1-3}$alkyl, $R_2$ is $R_2''$, $R_3$ is $R_3''$, $R_4$ is $R_4''$, $R_5$ is $R_5''$, $R_6$ is $R_6''$, especially hydrogen, $R_7$ is $R_7''$, and X is X'',

(xxvii)—(xxxii) of (xxi)—(xxvi) wherein any salt is a sodium, potassium or ammonium salt,

(xxxiii) of Group IBb wherein $R_1$ is $R_1'$, $R_2$ is $R_2'$, $R_3$ is $R_3'$, $R_4$ is $R_4'$, $R_5$ is $R_5'$, $R_{5a}$ is $R_{5a}'$, $R_6$ is $R_6'$ and X is X',

(xxxiv) of (xxxiii) wherein when $R_2'$ is other than hydrogen and $R_3'$ is hydrogen, $R_2'$ is in the 4-, 5- or 6-position; when both $R_2'$ and $R_3'$ are other than hydrogen, at least one of them is in the 5- or 6-position and neither of them is in the 7-position; when both $R_4'$ and $R_5'$ are other than hydrogen and $R_{5a}'$ is hydrogen, at least one of $R_4'$ and $R_5'$ is in a *meta* or *para* position; and when each of $R_4'$, $R_5'$ and $R_{5a}'$ is other than hydrogen, at least two of them are in *meta* or *para* positions,

(xxxv)—(xxxvi) of (xxxiii) and (xxxiv) wherein $R_6$ is $R_6''$, especially hydrogen,

(xxxvii)—(xxxviii) of (xxxiii) and (xxxiv) wherein $R_1$ is $C_{1-3}$alkyl, $R_2$ is $R_2''$, $R_3$ is $R_3''$, $R_4$ is $R_4''$, $R_5$ is $R_5''$, $R_6$ is $R_6''$, especially hydrogen, and X is X'',

(xxxix)—(lxiii) of (i)—(xiii) and (xxi)—(xxxii) wherein the hydroxy groups in the 3- and 5-positions (of the group of formula IIa) have the *erythro* configuration,

(lxiv)—(lxxxviii) the 3R,5S enantiomers of the compounds of (xxxix)—(lxiii) wherein X is

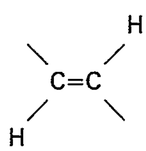

and the 3R,5R enantiomers of the compounds of these groups wherein X is $—(CH_2)_m—$,

(lxxxix)—(ci) of (xiv)—(xx) and (xxxiii)—(xxxviii) wherein the hydroxy group on the lactone ring IIb is *trans* to X (*i.e.*, the *trans* lactones), and

(cii)—(cxiv) the 4R,6S enantiomers of the compounds of (lxxxix)—(ci) wherein X is

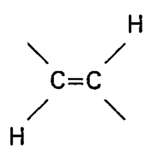

and the 4R,6R enantiomers of the compounds of these groups wherein X is $—(CH_2)_m—$.

Groups (xxxix)—(lxiii) embrace the 3R,5S—3S,5R racemate and the 3R,5S and 3S,5R enantiomers of the compounds wherein X is

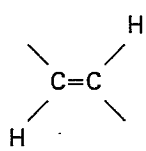

(the 3S,5R enantiomer being least preferred) and the 3R,5R—3S,5S racemate and the 3R,5R and 3S,5S enantiomers of the compounds wherein X is $—(CH_2)_m—$ (the 3S,5S enantiomer being least preferred).

Groups (lxxxix)—(ci) embrace the 4R,6S—4S,6R racemate and the 4R,6S and 4S,6R enantiomers of the compounds wherein X is

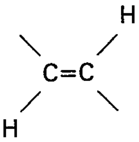

(the 4S,6R) enantiomer being least preferred) and the 4R,6R—4S,6S racemate and the 4R,6R and 4S,6S enantiomers of the compounds wherein X is $-(CH_2)_m-$ (the 4S,6S enantiomer being least preferred).

In the above-mentioned compound groups (i) to (cxiv) those containing a carboxyl group are preferably in salt form with a cation free from asymmetric carbon atoms e.g. sodium, potassium or ammonium, especially sodium.

A particular compound group covers those of formula I wherein

one of R and $R_0$ is

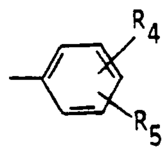

and the other is $C_{1-3}$alkyl, n-butyl or i-butyl,

wherein

$R_4$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy, and

$R_5$ is hydrogen, $C_{1-3}$alkyl, $C_{1-3}$alkoxy, trifluoromethyl, fluoro, chloro, phenoxy or benzyloxy, with the provisos that not more than one of $R_4$ and $R_5$ is trifluoromethyl, not more than one of $R_4$ and $R_5$ is phenoxy, and not more than one of $R_4$ and $R_5$ is benzyloxy,

$R_2$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that not more than one of $R_2$ and $R_3$ is trifluoromethyl, not more than one of $R_2$ and $R_3$ is phenoxy, and not more than one of $R_2$ and $R_3$ is benzyloxy,

X is $-(CH_2)_n-$ or

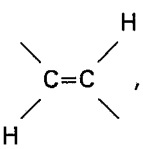 ,

wherein n is 0, 1, 2 or 3, and

Z is 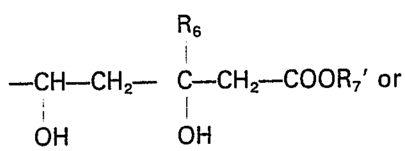 or 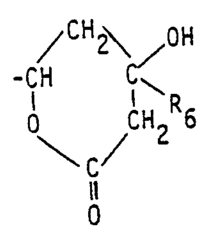

wherein

$R_6$ is hydrogen or $C_{1-3}$alkyl, and

$R_7'$ is hydrogen, $C_{1-3}$alkyl, n-butyl, i-butyl, t-butyl, benzyl or M, wherein M is a pharamceutically acceptable cation.

The compounds of formula I can be prepared by

a) when $R_6$ is hydrogen, reducing a compound of formula V

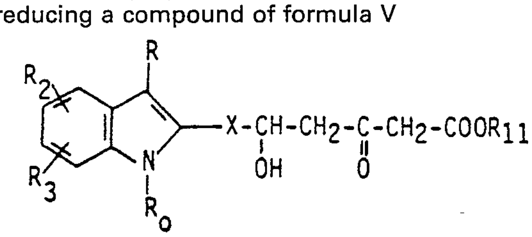

V

wherein $R_{11}$ is a radical forming a physiologically-hydrolysable and -acceptable ester and X, R, $R_0$, $R_2$ and $R_3$ are as defined above.

b) when $R_6 = C_{1-3}$alkyl, hydrolysing a compound of formula XII

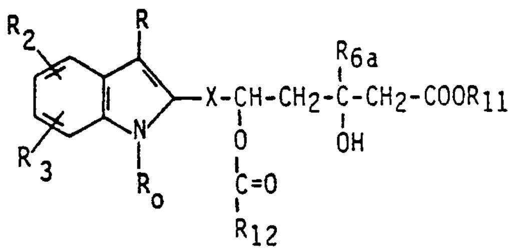

XII

wherein $R_{6a}$ is $C_{1-3}$alkyl, $R_{12}$ is an ester-forming group and X, R, $R_0$, $R_3$ and $R_{11}$ are as defined above,

c) when X is —CH=CH— deprotecting a compound of formula XXVIII

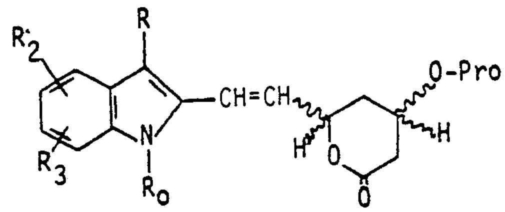

XXVIII

wherein Pro is a protecting group and R, $R_0$, $R_2$ and $R_3$ are as defined above,

d) hydrolysing a compound of formula I in the form of a physiologically-hydrolysable ester or a lactone or

e) esterifyig or lactonising a compound of formula I in free acid form, and when a free carboxyl group is present, recovering the compound obtained in free acid form or in the form of a salt. In processes a) and b) $R_{11}$ is preferably $C_{1-2}$alkyl, especially methyl, and $R_{12}$ is preferably $C_{1-3}$alkyl, especially $C_{1-2}$alkyl, in particular methyl.

It will readily be appreciated that the various forms of the compounds of formula I may interconverted as indicated in d) and e) above.

In the same way compounds obtained according to a), b), and c) may be hydrolysed to free acid forms and free acids forms may be esterified or lactonised to produce a desired end-product. The invention thus also provides a process for preparing a compound of formula I which comprises hydrolysing a compound of formula I in ester or lactone form or esterifying or lactonising a compound of formula I in free acid form and when a free carboxyl group is present recovering the compound obtained in free acid form or in the form of a salt.

Unless otherwise stated reactions are performed in a manner conventional for the type of reaction involved. Molar ratios and reaction times are as a rule conventional and non-critical and are chosen according to principles well established in the art on the basis of reactants and conditions employed.

Solvents, alone or as mixtures, are generally chosen which remain inert and liquid during the reaction in question.

Examples of inert atmospheres are carbon dioxide and more usually nitrogen or a nobel gas, nitrogen being preferred. Most reactions, including those wherein use of an inert atmosphere is not mentioned, are carried out under such for convenience.

Reduction according to a) is preferably carried out using a mild reducing agent such as sodium borohydride or, preferably, a complex or t-butylamine and borane in an inert organic solvent such as a lower alkanol, preferably ethanol, conveniently at a temperature of −10° to 30°, under an inert atmosphere.

Use of an optically pure starting material will lead to only two optical isomers (diastereoisomers) of the resulting end product. However, if stereospecificity is desired it is preferred to utilize a stereoselective reduction in order to maximize production of a mixture of the *erythro* stereoisomers (racemate) of which the preferred stereoisomer (as set forth above) is a constituent. Stereoselective reduction is preferably carried out in three steps. For example in the first step, the ketoester of formula V is treated with a tri(primary or secondary $C_{2-4}$alkyl)borane, preferably triethylborane or tri-n-butylborane, and air to form a complex. The reaction temperature is suitably 0° to 50°C, preferably 20° to 30°C. The first step is carried out in an anhydrous inert organic solvent, preferably an ether solvent such as tetrahydrofuran, diethyl ether, 1,2-dimethoxyethane or 1,2-diethoxyethane, with tetrahydrofuran being the most preferred solvent. In the second step, for example, the complex is reduced with sodium borohydride, preferably in the same solvent as utilized for the first step, at −100° to −40°C, preferably −90° to −70°C. in the third step, the product of the second step is, for example, treated with, preferably, anhydrous methanol at 20° to 40°C, preferably 20° to 30°C. The amount of methanol is not critical. However, a large excess, *e.g.,* 50—500 moles per mole of ketoester of formula V, is typically utilized.

Hydrolysis according to b) or d) is carried out in a manner conventional for such reactions e.g. employing an inorganic hydroxide such as NaOH or KOH with, if desired, subsequent acidification to give the free acid form. Suitable solvents are mixtures of water and water-miscible solvents such as lower alkanols e.g. methanol or ethanol and reaction conveniently takes place at temperatures from 20°C to reflux, preferably not more than 80°C. If it is desired to recover the compound in a salt form corresponding to the cation of the hydroxide employed then slightly less than equivalent amounts of the latter may be employed. In b) $R_{12}$ will conveniently be the same as $R_{11}$ e.g. $C_{1-3}$alkyl, especially $C_{1-2}$alkyl, preferably methyl.

Lactonisation according to e) is carried out in conventional manner e.g. by heating the corresponding acid in an anhydrous inert organic solvent e.g. a hydrocarbon such as benzene, toluene or a xylene or mixtures thereof, preferably at temperatures of 75°C to relfux although more preferably not above 150°C. Preferably, however, a lactonisation agent, e.g. a carbodiimide, preferably a water-soluble carbodiimide such as N-cyclohexyl-N'-[2-(N''-methylmorpholinium)ethyl]carbodiimide *p*-toluenesulfonate, in an anhydrous inert organic solvent, *e.g.,* a halogenated lower alkane, preferably methylene chloride, is employed. Reaction temperatures then lie typically between 10° and 35°C, especially 20° to 30°C.

As is evident to those in the art, a racemic *threo* 3,5-dihydroxycarboxylic acid yields a racemic *cis* lactone and a racemic *erythro* 3,5-dihydroxycarboxylic acid yields a racemic *trans* lactone. Use of a mixture of *threo* and *erythro* 3,5-dicarboxylic acid yields a mixture of *cis* and *trans* lactones (all four possible diastereoisomers). Likewise, if a single enantiomer of the 3,5-dihydroxycarboxylic acid is utilized, a single enantiomer of the lactone is obtained. For example, lactonisation of a 3R,5S *erythro*-3,5-dihydroxycarboxylic acid yields a 4R,6S lactone.

Esterification according to e) is conventional employing e.g. a large excess of a compound $R_{11}OH$, wherein $R_{11}$ is as defined above, at 20°C to 40°C in the presence of a catalytic amount of an acid such as *p*-toluenesulfonic acid. Where methyl esters are required these can also be obtained e.g. using diazomethane in an anhydrous inert ether solvent such as tetrahydrofuran, 1,2-dimethoxyethane or 1,2-diethoxyethane and especially diethylether at e.g. 0° to 30°C, preferably 20° to 30°C.

Examples of protecting groups in reaction c) are diphenyl-t-butylsilyl, tri-isopropylsilyl, dimethyl-t-butylsilyl, $C_{1-6}$n-alkyl, benzyl, triphenylmethyl, tetrahydrofuran-2-yl, tetrahydropyran-2-yl, 4-methoxy-tetrahydorpyran-2-yl and $C_{1-6}$n-alkanoyloxy. Especially preferred are trisubstituted silyl radicals, in particular diphenyl-t-butylsilyl.

Deprotection is carried out in conventional manner e.g. by cleavage under mild conditions such as employing e.g. for removal of a silyl group such as diphenyl-t-butylsilyl a fluoride reagent e.g. tetra-n-butylammonium fluoride in an anhydrous inert organic medium, preferably tetrahydrofuran containing glacial acetic acid at temperatures of 20° to 60°C, especially 20° to 30°C. Preferably 1—4 moles of fluoride are used per mole of silyl group with 1.2 to 1.8 moles of glacial acetic acid to each mole of fluoride.

The required starting materials may be prepared for example as illustrated in the following reaction schemes. The symbols used are defined as follows:

R, $R_0$, $R_1$, $R_2$, $R_3$, $R_4$, $R_5$, $R_{5a}$, $R_6$, $R_{6a}$, $R_{11}$, $R_{12}$, X = as defined above,
$R_{12a}$ = $C_{1-3}$alkyl, preferably $C_{1-2}$alkyl
Y = halogen, preferably chloro or bromo, more preferably chloro
Ac = acetyl
φ = phenyl
+ = t-butyl
lac =

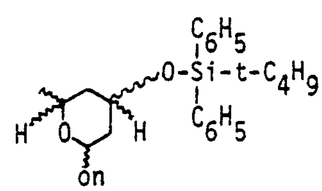

[on=$OCH_3$(XXVI), OH(XXVII) or =O(XXVIII)]

REACTION SCHEME I

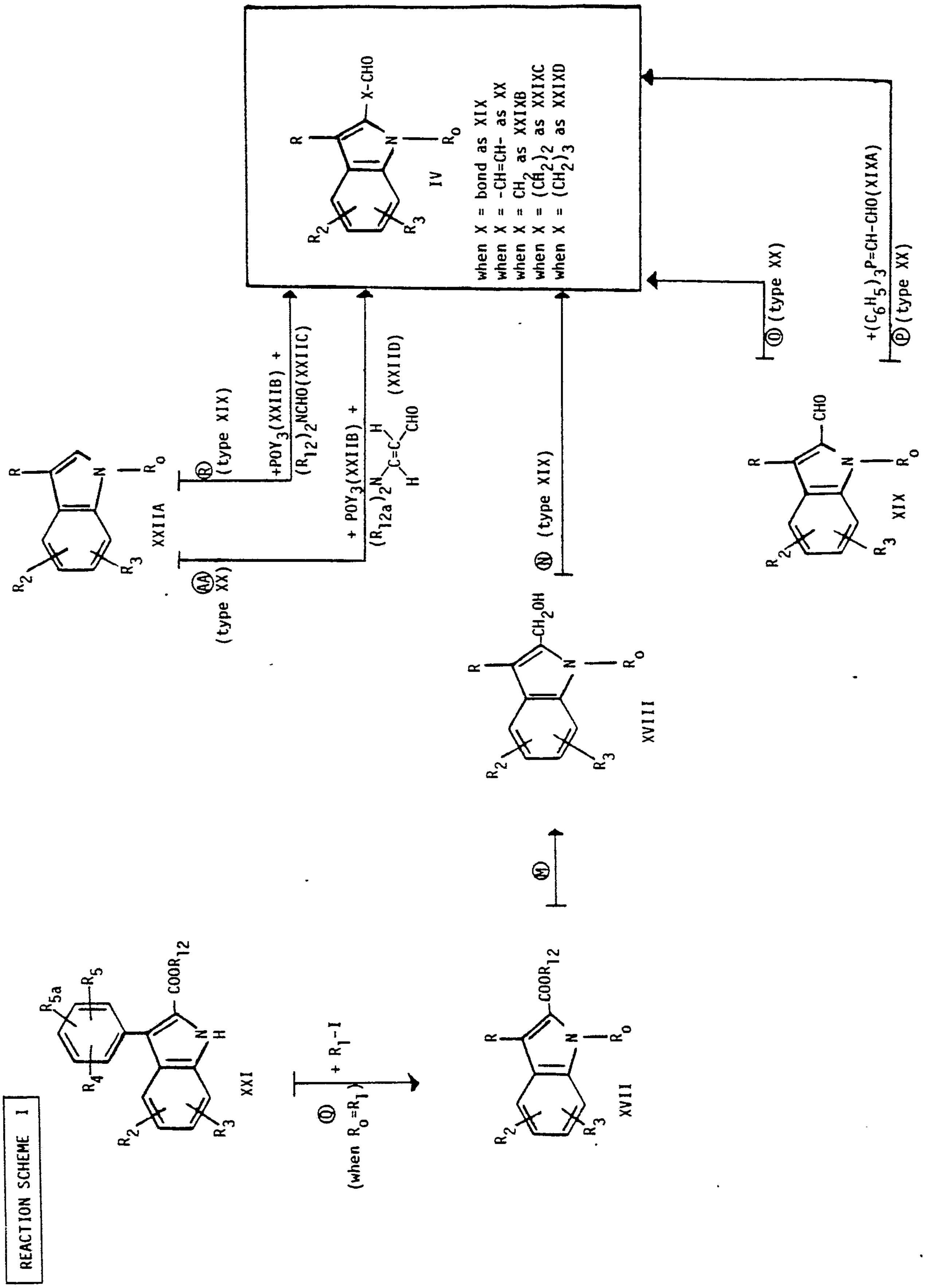

REACTION SCHEME II

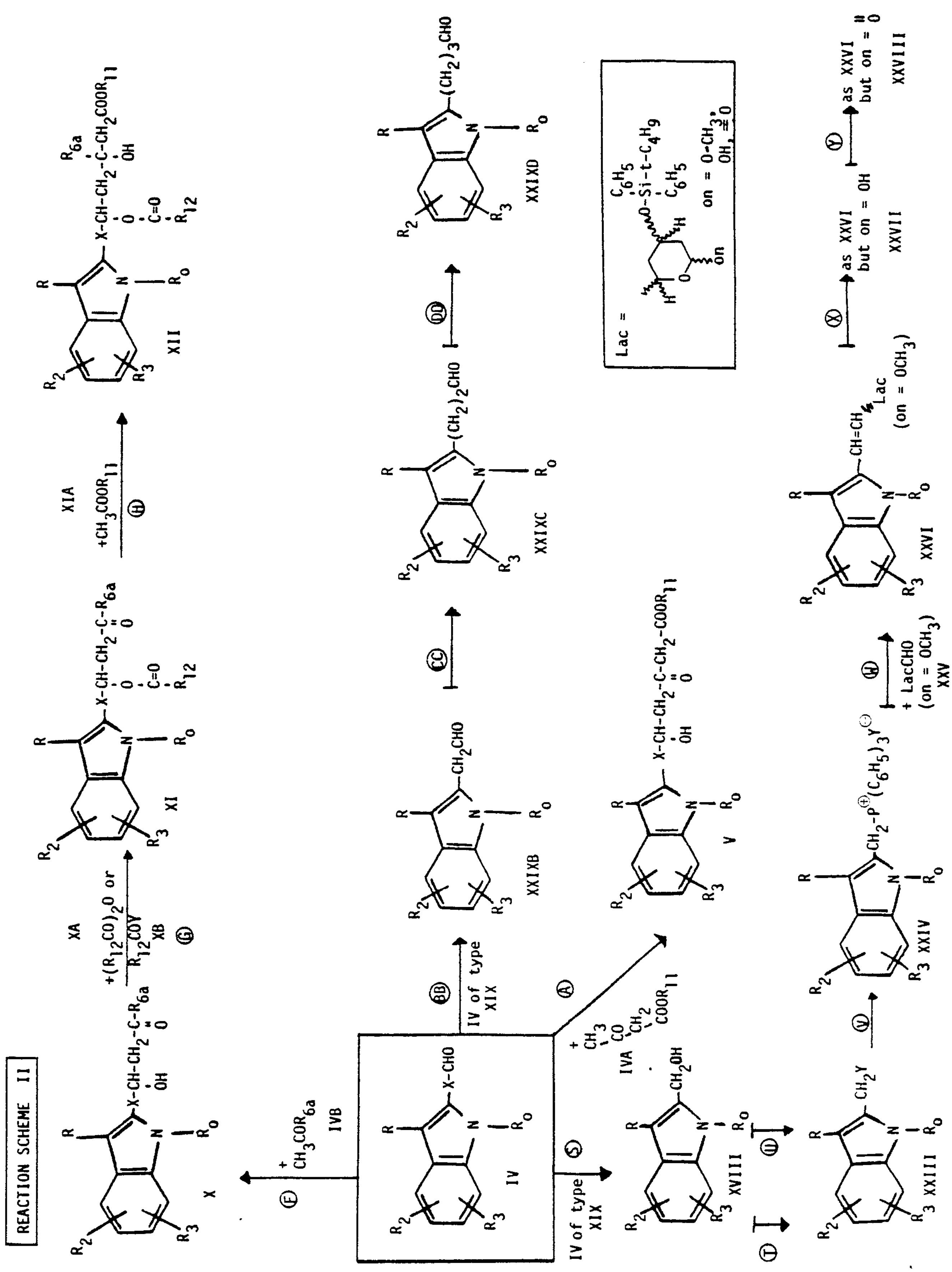

Reaction Scheme III

Two isomers of the compound of formula XXV may be synthesized by the following series of reactions:

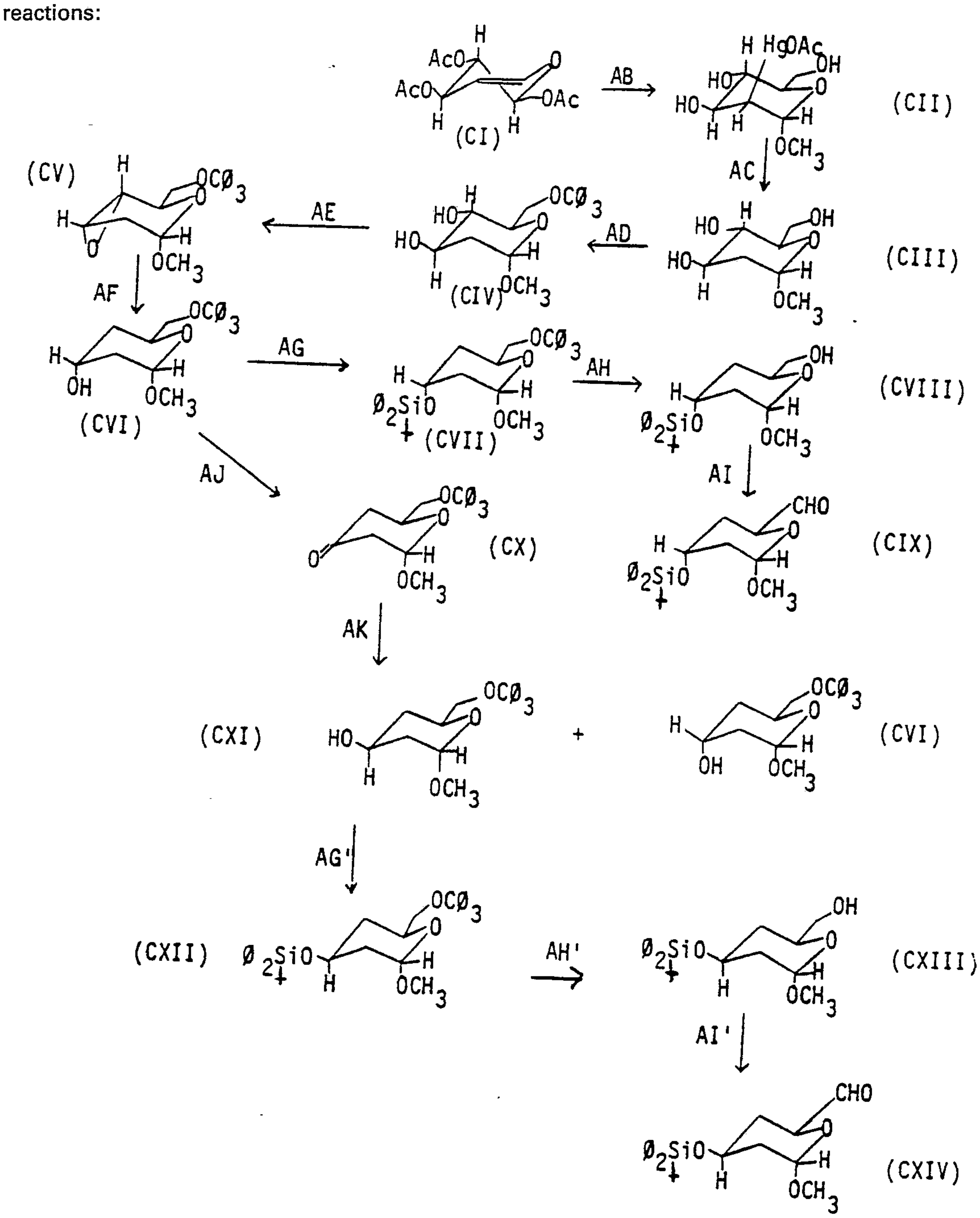


(CI) (CII) (CIII) (CIV) (CV) (CVI) (CVII) (CVIII) (CIX) (CX) (CXI) + (CVI) (CXII) (CXIII) (CXIV)

Unless otherwise stated reactions are performed in a manner conventional for the type of reaction involved. Mol ratios and reaction times are as a rule conventional and non-critical and are chosen according to principles well established in the art on the basis of reactants and conditions employed.

Solvents, alone or as mixtures, are generally chosen which remain inert and lqiuid during the reaction in question.

Examples of inert atmospheres are carbon dioxide and more usually nitrogen, helium, neon, argon or krypton, nitrogen being preferred. Most reactions, including those wherein use of an inert atmosphere is not mentioned are carried out under such for convenience.

The following tables give examples of typical reaction conditions in Reaction Schemes I and II temperatures being in degrees centigrade.

Abbreviations

THF (tetrahydrofuran)

DMF (dimethylformamide)

| Reaction | Type/Steps | Special conditions/Reagents | Temperatures | Atmosphere | Solvents |
|---|---|---|---|---|---|
| Ⓐ | 1. IVA + base<br>2. add IV | 1. Strong base e.g. n-butyl lithium for dianion, lithium diisopropylamde and NaH (latter for generation of mono-anion only). pref. Nah for monoanion then nBuLi for dianion | −80° to 10°<br>esp. −20° to 5° | inert gas | anhydrous THF, diethylether, 1,2-dimethoxy-, 1,2-diethoxy-ethane |
| Ⓕ | 1. IVB + base<br>2. add IV | 1. Strong base as Ⓐ preferred lithium diisopropylamide. Mol ratio of IVB to IV esp. 3:1 | −80° to −40°<br>esp. −80° to −75° | inert gas | as Ⓐ |
| Ⓖ | acylation | base e.g. pyridine, tertiary amine such as trimethylamine, 4-dimethyl-aminopyridine | 10° to 50°<br>esp. 20° to 30° | inert gas | excess pyridine and/or ether e.g. THF |
| Ⓗ<br>(cf. Ⓕ) | 1. XIA + base<br>2. add XI | reagents as Ⓐ<br>Mol ratio of XIA to XI<br>esp. 3:1 | 1. −80° to 0°<br>2. −80° to −40°<br>esp. −80° to −70° | inert gas | as Ⓐ<br>esp. THF |
| Ⓜ | reduction | metal hydride e.g. lithium aluminium hydride diisobutylaluminium hydride | −80° to reflux<br>pref. <70°<br>esp. −80° to 25° | | as Ⓐ<br>esp. THF |
| Ⓝ | mild oxidation | e.g. $MnO_2$ in large molar excess | 20° to reflux pref. <80° | | as Ⓐ |
| Ⓞ | 1. cis-1-lithium-2-ethoxyethylene*<br>2. p-TsOH<br><br>* prepared e.g. as described in the examples | 1.<br>2. p-toluenesulfonic acid in catalytic amount | 1. −80° to −40°<br>esp. −80° to −70° | 1. inert gas | 1. anhydr. THF<br>2. THF + water |

| Reaction | Type/Steps | Special conditions/Reagents | Temperatures | Atmosphere | Solvents |
|---|---|---|---|---|---|
| Ⓟ | Wittig | those usual for Wittig reactions | −20° to reflux pref. <140° | inert gas | as Ⓐ or anhyd. hydro-carbon e.g. benzene, toluene |
| Ⓠ | 1. XXI + base<br>2. $R_1$—I | 1. base e.g. as Ⓐ esp. NaH | 1. −20° to 30°<br>2. 15° to reflux pref. <80° esp. 20—30° | inert gas | as Ⓐ or anhydr. diethyl-acetamide and esp. dimethyl acetamide |
| Ⓡ** | Vilsmeir-Haack<br>1. XXIIB + XXIIC<br>2. XXIIA + iminium salt from 1<br>3. add base | 1. Phosphorous oxychloride is pref. as XXIIB<br>3. aqueous NaOH, KOH | 1. 0° to 35° esp. 0° to 15°<br>2. 60° to 120° esp. 80° to 105°<br>3. 10° to 45° esp. 10°—15° | 1. inert gas<br>2. inert gas | 1. and 2. anhydr. excess XXIIC alone or with lower alkyl-nitrile e.g. acetonitrile |
| Ⓢ | reduction | as Ⓜ pref. $NaBH_4$ | −5° to 35° | inert gas | as Ⓐ or pref. when using $NaBH_4$ ether + lower alkanol e.g. THF + methanol |
| Ⓣ | halogenation | phosphorous trihalide, thionyl-halide or oxalyl halide (halide = Y) + optionally a trace of DMF | 20° to 35° for phosphorous trihalide, otherwise −10° to 20° esp. −10° to 5° | inert gas | diethylether or THF |
| Ⓤ | halogenation | triphenylphosphine + $CY_4$ | −10° to reflux pref. <150° when run in excess $CY_4$ 25° to reflux pref. <100° | | excess $CY_4$ or as Ⓟ |

** Mol ratio 1. 1—5 of XXIIB + 1 of XXIIC: 1 of XXIIA
3. >4, pref. 4 to 6, of base per 1 of XXIIB in first step

| Reaction | Type/Steps | Special conditions/Reagents | Temperatures | Atmosphere | Solvents |
|---|---|---|---|---|---|
| Ⓥ | | triphenylphosphine | 60° to reflux pref. <150° esp. 100°—110° | inert gas | anhydr. hydrocarbon e.g. benzene, toluene or a xylene |
| Ⓦ | Wittig Reaction<br>1. base<br>2. XXV | 1. as Ⓐ<br>2. produces mixture of cis(Z) and trans(E) which can be separated here (conventionally) or further reacted and separated at a later stage | 1. 0° to 25°<br>2. −10° to reflux pref. <80° esp. 20° to 30° | inert gas | 1. + 2. as Ⓐ |
| Ⓧ | hydrolysis | acid e.g. 10% HCl in THF or glacial $CH_3COOH$ in THF/$H_2O$. Reaction produces some "6"-epimerisation which is greater than if HCl is used (pref. is glacial $CH_3COOH$/THF/$H_2O$: 3/2/1) | 10° to 100° esp. 60° | | THF with water |
| Ⓨ | oxidation (cf. Tetrahedron Letters *1976*. pp. 2503—6) | a) mild conditions e.g. N-methyl-morpholine oxide + dichlorotris-(($C_6H_5)_3P$)ruthenium II or e.g.<br>b) $Ag_2CO_3$ on celite | a) 0° to 40° pref. 20° to 30°<br>b) 0° to reflux pref. <150° esp. 100° to reflux | inert gas | a) anhydrous DMF, acetone<br>b) as Ⓥ |
| (AA) | cf. Ⓡ<br>1. XXIIB + XXIID<br>2. XXIIA + iminium salt from 1.<br>3. add base | cf. Ⓡ | 1. 210° to 25° esp. −10° to 5°<br>2. 60° to 100° esp. 65° to 85°<br>3. 20° to 45° esp. 20° to 30° | inert gas | as Ⓡ |

| Reaction | Type/Steps | Special conditions/Reagents | Temperatures | Atmosphere | Solvents |
|---|---|---|---|---|---|
| (BB)<br>(CC)<br>(DD) | Wittig | Successive Wittig reactions as (P) employing e.g. $(C_6H_5)_3P^{\oplus}—CH_2—OCH_3\ Cl^{\ominus}$ (XXIXA) e.g. firstly XXIXA + strong base secondly add aldehyde thirdly hydrolyse | cf. (P) | cf. (P) | cf. (P) |

CI is the commercially available compound tri-O-acetyl-D-glucal.

The preferred reactions conditions for Reactions AB—AI are:

AB: (1) sodium, methanol, 20°C, 15 minutes; (2) mercuric acetate, 25°C.

AC: sodium chloride, sodium borohydride, methanol + isopropanol, 20°C.

AD: triphenylmethyl chloride, pyridine, 35°C.

AE: (1) sodium hydride, tetrahydrofuran, 20°C, (2) 1-(2′,4′,6′-triisopropylbenzenesulfonyl)imidazole, −30°—20°C.

AF: lithium aluminium hydride, methyl *t*-butyl ether, −10°C.

AG: *t*-butyldiphenylchlorosilane, imidazole, N,N-dimethylformamide, 20°C.

AH: 70% aqueous trifluoroacetic acid, methylene chloride, −80° to −50°C especially −55°C rising over 1 hour to −10° to +10° esp. to −10° to 0°, and keeping at latter for 3—5 hours Epimerisation can be minimized by employing low temperatures and/or short times and terminating the reaction before completion.

AI: pyridinium chlorochromate or especially chromium trioxide (e.g. as Collins oxidation) in molar excess (e.g. 6 moles per mole of CVIII)/pyridine, pyridine, methylene chloride, 20°—30°C.

AJ: oxidation cf. AI.

AK: reduction cf. a) and S above especially $NaBH_4$.

Resulting compounds may be conventionally separated (e.g. HPLC or column chromatography) or directly further reacted.

The compounds of formulae IVA, IVB, XA, XB, XIA, XVII, XIXA, XXI, XXIIA—XXIID, XXIXA and CI and the reagents not designated by a Roman numeral are known or, if unknown, may be synthesized by processes analogous to those described in the literature for similar known compounds. As for the compound of formula XXV, one isomer is disclosed in Yang et al., Tetrahedron Letters *23*, 4305—4308 (1982), and the synthesis of two other isomers is disclosed in Reaction Scheme III.

The isomer of Yang et al. and one isomer disclosed in Reaction Scheme III yield lactones having the 4R,6S configuration and, as a result of epimerization in Reaction X, such compounds having the 4R,6R configuration. Lactones having the 4S,6R and 4S,6S configuration may be obtained from the other isomer whose synthesis is disclosed in Reaction Scheme III.

The availability of these intermediates enables synthesis of optically pure end products.

Reaction products, both intermediate and final, can be isolated and purified in conventional manner whereby intermediates can where appropriate be employed directly in a subsequent reaction.

Mixtures of stereoisomers (*cis, trans* and optical) may be separated by conventional means at whatever stage of synthesis is appropriate. Such methods include re-crystalisation, chromatography, formation of esters with optically pure acids and alcohols or of amides and salts (cf also Sommer et al., J.A.C.S. *80*, 3271 (1958)) with subsequent reconversion under retention of optical purity. For example diastereoisomeric (−)-α-naphthylphenylmethylsilyl derivatives of a lactone type end product of formula I may be separated on a silica column having covalently bound L-phenylglycine (eluant *n*-hexane/acetate: 1/1).

Salts may be prepared in conventional manner from free acids, lactones and esters and vice-versa. Whilst all salts are covered by the invention pharmaceutically acceptable salts, especially sodium, potassium and ammonium, particularly sodium, salts are preferred.

The various forms of the compounds of formula I are by virtue of their interconvertability useful as intermediates in addition to the use set out below.

Also within the scope of this invention are the intermediates of formulae V, X, XI, XII, XX, XXIV, XXVI—XXVIII and XXIXB—XXIXD. The preferences for each variable are the same as those set forth for the compounds of formula I, with the preferred groups of such compounds including those that correspond to Groups (i)—(xiii) and (xxxix)—(lxxxviii) (for formulae V, X—XII, XX and XXIXB—XXIXD) and Groups (xiv)—(xx), (xxxiii)—(xxxviii) and (lxxxix)—(cxiv) for formulae XXVI—XXVIII) to the extent consistent therewith.

The compounds of formula I possess pharmacological activity; in particular they are inhibitors of 3-hydroxy-3-methyl-glutaryl coenzyme A (HMG—CoA) reductase and as a consequence inhibitors of cholesterol biosynthesis as demonstrated in the following three tests.

Test A: In Vitro Microsomal Assay of HMG—CoA Reductase Inhibition:

200 µl. aliquots (1.08—1.50 mg/ml) of rat liver microsomal suspensions, freshly prepared from male Spargue-Dawley rats (150—225 g body weight), in Buffer A with 10 mmol dithiothreitol are incubated with 10 µl test substance dissolved in dimethylacetamide and assayed for HMG—CoA reductase activity as described in Ackerman et al., J. Lipid Res. *18*, 408—413 (1977). In the assay the microsomes are the source of the HMG—CoA reductase enzyme which catalyses the reduction of HMG—CoA to mevalonate. The assay employs a chloroform extraction to separate the product, [$^{14}$C]mevalonolactone, formed by the HMG—CoA reductase reaction from the substrate, [$^{14}$C]HMG—CoA. [$^{3}$H]mevalonolactone is added as an internal reference. Inhibition of HMG—CoA reductase is calculated from the decrease in specific activity [$^{14}$C/$^{3}$H]mevalonate) of test groups compared to controls.

Test B: In Vitro Cell Culture Cholesterol Biosynthesis Screen:

The cell culture is prepared as follows: Stock monolayer cultures of the Fu5AH rat hepatoma cell line (originally obtained from G. Rothblat; see Rothblat, Lipids *9*, 526—535 (1974)) are routinely maintained in

Eagle's Minimum Essential Medium (EMEM) supplemented with 10% fetal bovine serum (FBS) in 75 $cm^2$ tissue culture flasks. For these studies, when the cultures reach confluence, they are removed by mild enzymatic treatment with 0.25% trypsin in Hanks' balanced salt solution (without calcium and magnesium). After centrifugation of the cell suspension and aspiration of the enzymatic solution, the cell pellet is resuspended in an appropriate volume of media for seeding into 60 mm tissue culture dishes. The cultures are incubated at 37°C in an atmosphere of high humidity and 5% carbon dioxide. When the cultures are confluent (approximately 5 days), they are ready for use. The culture media is aspirated from the dishes and replaced with 3 ml of EMEM supplemented with 5 mg/ml of dilipidized serum protein (DLSP) prepared by the method of Rothblat et al., In Vitro *12*, 554—557 (1976). Replacement of the FBS with DLSP has been shown to stimulate the incorporation of [$^{14}$C]acetate into sterol by removing the exogenous sterol supplied by the FBS, thereby requiring the cells to synthesize sterol. Enhanced 3-hydroxy-3-mthylglutaryl Coenzyme A reductase (HMG—CoA reductase) activity is measurable in the cells in response to the lack of exogenous sterol. Following approximately 24 hours incubation at 37°C in the DLSP supplemented media, the assay is initiated by the addition of 3μCi if [$^{14}$C]acetate and the test substances solubilized in dimethylsulfoxide (DMSO) or distilled water. Solvent controls and compactin-treated controls are always prepared. Triplicate 60 mm tissue culture dishes are run for each group. After 3 hours incubation at 37°C, the cultures are examined microscopically using an inverted phase contrast microscope. Notations are made of any morphological changes which may have occurred in the cultures. The media is aspirated and the cell layer is gently washed twice with 0.9% sodium chloride solution (saline). The cell layer is then harvested in 3 ml of 0.9% saline by gentle scraping with a rubber policeman and transferred to a clean glass tube with Teflon lined cap. The dishes are rinsed with 3 ml of 0.9% saline and rescrapped, and the cells are combined with the first harvest. The tubes are centrifuged at 1500 r.p.m. for 10 minutes in an IEC PR—J centrifuge, and the supernatant is aspirated.

The cells are then extracted as follows: One ml of 100% ethanol is added to the cell pellet followed by sonication for 10 seconds with a "LO" setting of 50 on a Bronwell Biosonik IV. One hundred μl are taken for protein determination. One ml of 15% potassium hydroxide (KOH) is added, and the samples are thoroughly vortexed. Saponification is accomplished by heating the ethanol-KOH treated samples at 60°C for 60 minutes in a water bath. Following dilution of the samples with 2 ml of distilled water, they are extracted three times with 7 ml of petroleum ether. The petroleum ether extracts are then washed three times with 2 ml of distilled water and finally taken to dryness under a stream of nitrogen.

The obtained samples are then analyzed by thin layer chromatography (TLC) as follows: Residues from the petroleum ether extraction are taken up in a small volume of hexane and spotted on silica gel 60 TLC plates (E. Merck). Development of the plates is carried out in a three phase solvent system consisting of 150 parts by volume hexane: 50 parts by volume diethyl ether: 5 parts by volume glacial acetic acid, visualization is accomplished in an iodine vapor chamber. The plates are divided into five sections such that each section contains the molecules having the following approximate Rf values: section 1- 0—0.4, section 2- 0.4—0.55, section 3- 0.55—0.7, section 4- 0.7—0.9 and section 5- 0.9—1.0. Section 2 contains the non-saponifiable sterols. The five sections of the TLC plates are scraped into scintillatin vials. Blanks are also prepared from scrapings of chromatographed non-labelled standards. ASC® scintillation cocktail is added, and the radioactivity is determined in a liquid scintillation spectrometer. [$^{14}$C]hexadecene standards are used to determined counting efficiencies. The total protein content of the samples is determined employing the Bio-Rad Protein Assay System.

The results are reported as distintegrations per minute per mg protein (d.p.m./mg protein) for each of the five TLC sections. Mean d.p.m./mg protein ± standard error of the mean are compared for percentage change (%$\Delta$) and statistical significance with solvent control means. TLC section 2 data is taken as a measure of HMG—CoA reductase activity inhibition.

Test C: In Vivo Cholesterol Biosynthesis Inhibition Tests: In vivo studies utilize male Wistar Royal Hart rats weighing 150 ± 20 g which have been kept for 7—10 days on an altered light cycle (6:30 a.m—6.30 p.m. dark) housed two per cage and fed powdered Purina Rat Chow and water — *ad libitum*. Three hours before the diurnal maximum of cholesterol synthesis at mid-dark, the rats are administered the test substance dissolved or as a suspension in 0.5% carboxymethylcellulose in a volume of 1 ml/100 g body weight. Controls receive vehicle alone. One hour after receiving the test substance, the rats are injected intraperitoneally with about 25 μCi/100 g body weight of sodium [1-$^{14}$C]acetate 1—3 mCi/mmol. Two hours after mid-dark, blood samples are obtained under sodium hexobarbitol anesthesia and the serum separated by centrifugation.

Serum samples are saponified and neutralized, and the 3β-hyroxysterols are precipitated with digitonin basically as described by Sperry et al., J. Biol. Chem. *187*, 97 (1950). The [$^{14}$C]digitonides are then counted by liquid scintillation spectrometry. After correcting for efficiences, the results are calculated in nCi (nanocuries) of sterol formed per 100 ml of serum. Inhibition of sterol synthesis is calculated from the reduction in the nCi of sterols formed from test groups compared to controls.

The compounds are thus indicated for use as hypolipoproteinemic and anti-antherosclerotic agents.

An indicated suitable daily dosage for use in the treatment of hyperlipoproteinemia and atherosclerosis is from about 1 to 2000 mg, preferably 1.5 to 100 mg, suitably administered in divided dosages of 0.25 to 1000 mg, preferably 0.4 to 50 mg two to four times daily or in retard form.

They may be administered in free acid form or in the form of a physiologically-hydrolysable and

-acceptable ester or a lactone thereof or in pharmaceutically acceptable salt form whereby the various forms have activities in the same range.

The invention therefore also concerns compounds of formula I in free acid form or in the form of a physiologically-hydrolysable and -acceptable ester or a lactone thereof or in pharmaceutically acceptable salt form for use as pharmaceuticals e.g. as hypolipoproteinemic and anti-atherosclerotic agents.

The compounds may be administred alone, or in admixture with a pharmaceutically acceptable diluent or carrier, and, optionally, other excipients, and administered orally in such forms as tablets, elixirs, capsules or suspensions or parenterally in such forms as injectable solutions or suspensions.

The preferred pharmaceutical compositions from the standpoint of ease of preparation and administration are solid compositions, particulary tablets and hard-filled or liquid-filled capsules.

Such compositions also form part of the invention.

The following examples, in which all temperatures are in °C, illustrate the invention.

Example 1

Methyl (E)-3,5-dihydroxy-7-[3′-(4′′-fluorophenyl)-1′-methylindol-2′-yl]hept-6-enoate (Compound No. 1)

a) Ethyl 3-(4′-fluorophenyl)-1-methylindole-2-carboxylate (Reaction Q; compound XVIIa)

To a solution of 8.0 g (28 mmol) of ethyl 3-(4′-fluorophenyl)indole-2-carboxylate in 30 ml of dry dimethylacetamide stirred under nitrogen at −10°, 1.6 g (33 mmol) of sodium hydride is added. The reaction mixture is stirred at −10° under nitrogen for 45 minutes, 4.8 g (32 mmol) of methyl iodide is added at −10°, and the reaction mixture is allowed to warm to room temperature and stirred under nitrogen at room temperature for 2 hours. The reaction mixture is poured into 400 ml of ice/water, neutralized with 4 ml of 2N hydrochloric acid and extracted several times with diethyl ether. The diethyl ether extracts are combined, washed with water, washed with saturated sodium chloride solution, dried over anhydrous magnesium sulfate, filtered and evaporated at reduced pressure. The residue is purified by column chromatography utilizing a silica gel column and chloroform as the eluant. The fractions containing the product are combined and evaporated at reduced pressure, and the residue is crystallized from *n*-hexane/petroleum ether to obtain the product, m.p. 61—62°.

b) 3-(4′-Fluorophenyl)-2-hydroxymethyl-1-methylindole (Reaction M; compound XVIIIa)

To a solution of 20.0 g (67 mmol) of compound XVIIa in 500 ml of dry tetrahydrofuran stirred at −78° under nitrogen, 80 ml of 25% (by weight) diisobutylaluminum hydride/toluene is added, and the reaction mixture is stirred at −78° under nitrogen for 4 hours. The reaction mixture is allowed to warm to −10°, an additional 30 ml of 25% (by weight) diisobutylaluminum hydride/toluene is added, the reaction mixture is stirred at 0° under nitrogen for an additional 3 hours, a further 30 ml of 25% (by weight) diisobutylaluminum hydride/toluene is added, and the reaction mixture is stirred at 0° under nitrogen for a further 1 hour. The reaction mixture is treated with saturated ammonium chloride solution and filtered, and the organic layer is separated, dried over anhydrous magnesium sulfate, filtered and evaporated at reduced pressure. The residue is triturated with *n*-hexane to obtain the product, m.p. 99°—104°.

c) 3-(4′-Fluorophenyl)-1-methylindole-2-carboxaldehyde (Reaction N; compound IVa)

A mixture of 17.0 g (67 mmol) of compound XVIIIa, 90.0 g (1.03 mol) of manganese dioxide and 1.2 l of anhydrous diethyl ether is stirred at room temperature under nitrogen for 14 hours. The reaction mixture is filtered, and the diethyl ether is evaporated at reduced pressure. The residue is flash chromatographed on a silica gel column using methylene chloride as the eluant, the fractions containing the product are combined and evaporated at reduced pressure, and the residue is triturated with *n*-pentane to obtain the product, m.p. 75°—79°.

d) (E)-3-[3′-(4′′-Fluorophenyl)-1′-methylindol-2′-yl]propenaldehyde (Reaction O; compound IVb)

25 ml of 1.7 M. *n*-butyl lithium/*n*-hexane (42 mmol) is added dropwise to a solution of 14.5 g (40 mmol) of tri-*n*-butylstannylvinylethoxide in 600 ml of dry tetrahydrofuran stirred at −78° under nitrogen, stirring is maintained for 2 hours under the same conditions, and 9.0 g (35.6 mmol) of compound IVa dissolved in 60 ml of dry tetrahydrofuran is added rapidly dropwise. The reaction mixture is stirred at −78° under nitrogen for 3.5 hours, quenched with 60 ml of saturated ammonium chloride solution and extracted several times with diethyl ether. The diethyl ether extracts are combined, washed with water, washed with saturated sodium chloride solution, dried over anhydrous magnesium sulfate, filtered and evaporated at reduced pressure. The residue is partitioned between *n*-hexane and acetonitrile (to remove the organotin compounds), and the acetonitrile layer is evaporated at reduced pressure to obtain an oil. The oil is dissolved in 300 ml of tetrahydrofuran, 50 ml of water and 30 mg of *p*-toluenesulfonic acid monohydrate are added, and the reaction mixture is stirred for 2 hours at room temperature and then extracted several times with diethyl ether. The diethyl ether extracts are combined, washed with water, washed with saturated sodium chloride solution, dried over anhydrous magnesium sulfate, filtered and evaporated to dryness at reduced pressure. The residue is triturated with *n*-hexane/diethyl ether to obtain the product, m.p. 110°—112°. A subsequent batch melted at 115—118°.

e) Methyl (E)-7-[3'-(4''-fluorophenyl-1'-methylindol-2'-yl]-5-hydroxy-3-oxohept-6-enoate (Reaction A; compound Va)

3.5 ml (32.4 mmol) of methyl acetoacetate is added dropwise to a suspension of 1.6 g (by weight) sodium hydride (33.3 mmol) in 400 ml of dry tetrahydrofuran stirred at −15° under nitrogen. The reaction mixture is stirred at −15° under nitrogen for 20 minutes, 19 ml of 1.7 M. *n*-butyllithium/*n*-hexane (31.9 mmol) is added, the reaction mixture is stirred at −15° under nitrogen for 20 minutes, a solution of 5.3 g (19 mmol) of compound IVb in 100 ml of dry tetrahydrofuran is added, and the reaction mixture is stirred at −15° under nitrogen for 30 minutes. The reaction mixture is quenched with dilute hydrochloric acid and extracted several times with diethyl ether. The diethyl ether extracts are combined, washed with water, washed with saturated sodium chloride solution, dried over anhydrous magnesium sulfate, filtered and evaporated to dryness at reduced pressure. The residue is triturated with *n*-pentane (to remove excess methyl acetoacetate) to obtain the crude product as an oil.

The product is a racemate that may be resolved into its d and l components.

f) Methyl (E)-3,5-dihydroxy-7-[3'-(4''-fluorophenyl)-1'-methylindol-2'-yl]hept-6-enoate (process a); Compound No. 1)

2.0 g of borane-t-butylamine complex is added to a solution of 8.0 g (20.2 mmol) of crude racemic compound Va in 200 ml of absolute ethanol stirred at 0° under nitrogen. The reaction mixture is stirred at 0° under nitrogen for 3 hours, and saturated sodium chloride solution is added. The reaction mixture is acidified with dilute hydrochloric acid and extracted several times with diethyl ether. The diethyl ether extracts are combined, washed with water, washed with saturated sodium chloride solution, dried over anhydrous magnesium sulfate, filtered and evaporated to dryness at reduced pressure. The obtained oil is purified by flash chromatography using a silica gel column and 1:1 ethyl acetate/chloroform as the eluant. The product, a mixture of four stereoisomers, is obtained as a yellow oil.

The obtained mixture of stereoisomers (*erythro* and *threo*) may be separated by conventional means into two racemic mixtures each of which may be resolved into two optically pure enantiomers. The four isomers may be designated as the 3R,5R, 3S,5S, 3R,5S and 3S,5R isomers. Preferred are the 3R,5R and 3R,5S isomers and the racemate of which each is a consistent, *viz.*, the 3R,5R-3S,5S and the 3R,5S-3S,5R racemate.

### Example 2

(E)-3,5-Dihydroxy-7-[3'-(4''-fluorophenyl)-1'-methylindo-2'-yl]-hept-6-enoic acid (process d)/ester hydrolysis; Compound No. 3)

2.8 ml of 1N aqueous potassium hydroxide (2.8 mmol) is added to a solution of 1.1 g (2.77 mmol) of Compound No. 1 in 100 ml of 95% aqueous methanol stirred at room temperature, and the reaction mixture is stirred at room temperature for 3 hours. The solvent is evaporated at reduced pressure, the residue [crude potassium salt (Compound 2) a mixture of four stereoisomers] is dissolved in water, and the aqueous solution is extracted with diethyl ether. The aqueous phase is acidified with dilute hydrochloric acid (pH 6.0) and extracted several times with diethyl ether. The diethyl ether extracts are combined, washed with water, washed with saturated sodium chloride solution, dried over anhydrous magnesium sulfate, filtered and evaporated at reduced pressure to give the crude product as a yellow oil. It is a mixture of four stereoisomers.

If desired, the potassium salt or free acid (Compound No. 3) may be separated into two racemic mixtures (*erythro, threo*) each of which may be resolved into two optically pure enantiomers. The four stereoisomers may be designated as the 3R,5R, 3S,5S, 3R,5S and 3S,5R isomers. Preferred are the 3R,5R and 3R,5S isomers and the racemate of which each is a constituent, *viz.,* the 3R,5R-3S,5S racemate and the 3R,5S-3S,5R racemate.

### Example 3

(E)-6-[3'-(4''-Fluorophenyl)-1'-methylindol-2'-ylethenyl]-4-hydroxy-3,4,5,6-tetrahydro-2H-pyran-2-one (process e)/lactonisation; compound 4)

a) A solution of 1.1 g (2.87 mmol) of crude compound no. 3 in 50 ml of dry benzene is refluxed for 8 hours. The solvent is evaporated at reduced pressure, and the residue is flash chromatographed on a silica gel column utilizing 19:1 chloroform/methanol as the eluant to obtain the product as a mixture of four diasteroisomers (two *cis* and two *trans*) (compound 4).

bi) The mixture of isomers obtained is separated by high pressure liquid chromatography using a silica gel column and, as the solvent, 7:2:1 methyl *t*-butyl ether/*n*-hexane/acetone to obtain the racemic *trans* compound, m.p. 147—150° (compound no. 5). A subsequent batch melted at 150—154°.

bii) The obtained *trans* racemate may be resolved by conventional means into two optically pure enantiomers, the 4R,6S and 4S,6R isomers by, for example, (i) reacting with (−)-α-naphthylphenylmethylchlorosilane, (ii) separating the obtained diastereoisomeric silyloxy compounds and (iii) cleaving the silyl groups with tetra-*n*-butylammonium fluoride in a mixture of acetic acid and tetrahydrofuran, as set forth above. The amorphous solid 4R,6S enantiomer has an $[\alpha]_D^{25} = -18.5°$ ($CHCl_3$, c = 0.2 g (compound 17). The 4S,6R enantiomer was also an amorphous solid.

c) The racemic *cis* lactone may also be isolated from the silica gel column, m.p. 48°—62°C (dec.)

(compound 6). It too may be resolved by conventional means into two optically pure enantiomers. The two stereoisomers may be designated at the 4R,6R and 4S,6S isomers, the former being preferred.

Example 4

Methyl (±)-*erythro*-(E)-3,5-dihydroxy-7-[3'(4''-fluorophenyl)-1'-(1''-methylethyl)indol-2'-yl]hept-6-enoate (Compound No. 7)

a) (E)-3-[3'-(4''-Fluorophenyl)-1'-(1''-methylethyl)indol-2'-yl]-prop-2-enal (Reaction AA; compound IVc)

A solution of 50 ml (49.6 g, 0.5 mole) of 3-N,N-dimethylaminoacrolein in 200 ml of dry acetonitrile is slowly added over a 30 minute period to a solution of 50 ml (82.5 g, 0.5392 mole) of phosphorus oxychloride in 200 ml of dry acetonitrile stirred at −10°—0° under nitrogen. 45.3 g (0.1788 mole) of 3-(4'-fluorophenyl-1-(1'-methylethyl)indole (XXIIAa) is added portionwise over a 2 minute period to the reaction mixture stirred at 0°—5°. The reaction mixture is refluxed for 24 hours under nitrogen, cooled to room temperature and slowly poured (over a 20 minute period) into a cold (10°) stirred mixture of 2 litres of toluene and a solution of 130 g of sodium hydroxide in 2 litres of water so that the temperature does not exceed 26°. The reaction mixture is filtered to remove the insolubles, and the toluene layer is separated and washed twice with 1 litre portions of water. The additional insolubles are removed by filtration, and the toluene layer is evaporated at reduced pressure and 50°—60°. The obtained viscous oil is chromatographed on 550 g of silica gel (20—230 mesh A.S.T.M.) using methylene chloride as the eluant; twenty 100 ml fractions are collected over a 2 hour period. The fractions containing the desired product (as determined by thin layer chromatography) are combined and evaporated to dryness at reduced pressure and 50°—60° to obtain the crude solid product. The crude product is dissolved in 70 ml of refluxing absolute ethanol, the obtained solution is cooled to 65°, 70 ml of *n*-heptane is added, and the resulting solution is cooled to −5°—0° for 15 minutes. The precipitated solids are collected by filtration, washed with 20 ml of ice cold *n*-heptane and vacuum dried at 50°—55° to obtain the yellow product, m.p. 122°—123°. A subsequent batch melted at 129—132°.

3-(4'-Fluorophenyl-1-(1'-methylethyl)indole (m.p. 94.5—95°) is prepared via 4-chloroacetyl-1-fluorobenzene and N-(4-fluorobenzoylmethyl)-N-(1-methylethyl)aniline (m.p. 78—81°) in conventional manner.

b) Methyl (±)-(E)-7-[3'-(4''-fluorophenyl)-1'-(1''-methylethyl)-indol-2'-yl]-5-hydroxy-3-oxohept-6-enoate (Reaction A, compound Vb)

Analogous to 1e) m.p. 95—97°.

The product is a racemate which may be resolved into its R and S components.

c) Methyl (±)-*erythro*-(E)-3,5-dihydroxy-7-[3'(4''-fluorophenyl)-1'-(1''-methylethyl)indol-2'yl]hept-6-enoate Process a)/stereospecific; compound no. 7)

(i) 30 ml of 1M triethylborane/tetrahydrofuran (30 mmol) is added dropwise to a solution of 12.2 g (26 mmol assuming 100% yield) of crude Compound Vb in 400 ml of dry tetrahydrofuran (distilled over lithium aluminium hydride) stirred at room temperature, 55 ml of air (at 760 mm Hg and 25°) is bubbled through over 5 minutes, and the reaction mixture is stirred at room temperature under nitrogen for 2 hours. The reaction mixture is cooled to −80°, 1.3 g (34 mmol) of sodium borohydride is added, and the reaction mixture is stirred overnight at −80° under nitrogen. The reaction mixture is allowed to warm to −10°—0°, quenched by the dropwise addition of sufficient 2N hydrochloric acid to lower the pH to 2 and extracted with diethyl ether. The diethyl ether extract is washed twice with saturated sodium chloride solution, dried over anhydrous magnesium sulfate and evaporated at reduced pressure to a yellow oil, the crude ethylborate ester. 400 ml of anhydrous methanol is added, and the reaction mixture is stirred at room temperature for 2.5 hours. The methanol is evaporated at reduced pressure and 40°, and the residue is dissolved in 4:1 (by volume) chloroform/ethyl acetate and chromatographed on a silica gel column using the same solvent as the eluant. The fractions containing the relatively pure product are combined and evaporated at reduced pressure to obtain the product as an oil.

(ii) An impure chromatography fraction (containing some product) is evaporated at reduced pressure, and the residue is triturated with diethyl ether and *n*-pentane and seeded with a crystal that formed upon addition of the methanol to the ethylborate ester to obtain the product (96% *erythro*) as a white powder, m.p. 122°—124°.

The product is a racemate which may be resolved into two optically pure enantiomers, the 3R,5S and 3S,5R isomers, of which the former is preferred.

Example 5

*Erythro*-(±)-(E)-3,5-dihydroxy-7-[3'-(4''-fluorophenyl)-1'-(1''-methylethyl)indol-2'-yl]hept-6-enoic acid (process d)/esterhydrolysis; compound no. 8)

(a) 4.5 ml of 1N sodium hydroxide solution (4.5 mmol) and 2.0 g (4.7 mmol) of Compound no. 7 are stirred in 150 ml of ethanol at room temperature for 2 hours, the solvent is evaporated at reduced pressure, and the residue is dissolved in 50 ml of water. The aqueous solution is gently extracted with diethyl ether, the traces of ether in the aqueous layer are removed at reduced pressure, and the aqueous layer is freeze dried to obtain the racemic product as its sodium salt (compound no. 9) m.p. 194°—197°.

(b) Compound no. 9 is dissolved in water, and the solution is acidified to pH 2 with 2N hydrochloric acid and extracted with diethyl ether. The diethyl ether extract is washed three times with saturated sodium chloride solution, dried over anhydrous magnesium sulfate and evaporated at reduced pressure to obtain the crude solid racemic free acid (compound no. 8).

The sodium salt and free acid can be resolved into their 3R,5S and 3S,5R isomers, the former being preferred.

Example 6

(E)-(±)-4-hydroxy-6-[3'-(4''-fluorophenyl)-1'-(1''-methylethyl)-indol-2'-ylethenyl]-3,4,5,6-tetrahydro-2H-pyran-2-one (process e)/catalyzed lactonisation; compound no. 10)

6.9 g of crude Compound no. 8 (which contains some *threo* isomer) and 7 g of N-cyclohexyl-N'-[2-(N''-methylmorpholinium)ethyl]carbodiimide *p*-toluenesulfonate are stirred in 300 ml of methylene chloride at room temperature for 3 hours. The reaction mixture is extracted with water, dried over anhydrous magnesium sulfate and evaporated at reduced pressure. The residual oil (containing compound no. 10) is chromatographed on a silica gel column utilizing 7:2:1 (by volume) methyl *t*-butyl ether:*n*-hexane:acetone as the eluant. The initial fractions, containing the racemic *trans* lactone, are combined and evaporated at reduced pressure to obtain the product as a foam (compound no. 11). Fractions contianing the *cis* compound are evaporated at reduced pressure to obtain a solid product, m.p. 170—175° (dec.) (compound no. 12).

The *trans* and *cis* products are racemates which may be resolved into their 4R,6S and 4S,6R or 4R,6R and 4S,6S isomers respectively, the former being preferred in each case.

Example 7

Sodium *erythro*-(±)-(E)-3,5-dihydroxy-7-[3'-(4''-fluorophenyl)-1'-(1''-methylethyl)indol-2'-yl]hept-6-enoate (alternative method/process d)/lactone hydrolysis; compound no. 13 = cmpd. 9)

2.6 g of the corresponding *trans* lactone (cf. Ex. 6), 12.6 ml of 0.5N sodium hydroxide solution (6.3 mmol) and 200 ml of absolute ethanol are stirred for 2 hours at room temperature, the solvent is evaporated at reduced pressure, and the residue is dissolved in 150 ml of water. The aqueous solution is gently washed with diethyl ether and freeze dried to obtain the solid racemic product. This compound can be resolved into its optically pure isomers 3R,5S ($[\alpha]_D^{25} = -13.33°$ ($CHCl_3$, c = 0.99 g); compound no. 14) and 3S,5R (compound no. 15), the former being preferred.

The 3R,5S isomer can also be obtained by the above process directly from the optically pure compound 20.

Example 8

Sodium *threo*-(±)-(E)-3,5-dihydroxy-7-[3'-(4''-fluorophenyl)-1'-(1''-methylethyl)indol-2'-yl]hept-6-enoate (compound no. 16)

Obtained analogously to Example 7 from the corresponding *cis* lactone.

This compound may also be resolved into its optically pure isomers 3R,5S and 3S,5R, whereby the former is preferred.

Example 9

(E)-*Trans*-6S-[3'-(4''-fluorophenyl)-1'-methylindol-2'-ylethenyl]-4R-hydroxy-3,4,5,6-tetrahydro-2H-pyran-2-one (compound no. 17)

a) 3-(4'-Fluorophenyl)-1-methylindole-2-carboxaldehyde (Reaction R; compound IVd)

78.5 ml (0.84 mole) of phosphorus oxychloride is added dropwise over a 20 minute period to 213 ml of dimethylformamide stirred at 0° under nitrogen, the temperature of the reaction mixture not being allowed to exceed 10°. The reaction mixture is heated to 80°, a solution of 163.5 g (0.727 mole) of 3-(4'-fluorophenyl)-1-methylindole in 270 ml of dimethylformamide is added at a rate such that the temperature of the reaction mixture is maintained at 81°—83°, the reaction mixture is maintained at 80°—81° for 5 hours and cooled to 10°, and 1 litre of 15% sodium hydroxide solution is added dropwise at a rate such that the temperature of the reaction mixture is maintained at 35°—40°, the reaction mixture being stirred under nitrogen throughout. The reaction mixture is cooled to 25°, and the solids are collected by filtration, washed three times with 500 ml portions of water and dissolved in 500 ml of methylene chloride. The methylene chloride solution is filtered through 500 ml of silica gel (70—230 mesh A.S.T.M.) and the silica gel is carefully washed with 2 litres of methylene chloride. The methylene chloride solutions are combined and concentrated to a volume of 300 ml at reduced pressure, 300 ml of absolute ethanol is added and the reaction mixture is distilled until the internal temperature reaches 78°. The reaction mixture is cooled to 0° and the precipitated bright yellow product is collected by filtration and vacuum dried at room temperature, m.p. 80.5°—81.5°.

b) 3-(4'-Fluorophenyl)-2-hydroxymethyl-1-methylindole (Reaction S; compound XVIIIa)

A solution of 160 g (0.6324 mole) of Compound IVd in 650 ml of tetrahydrofuran is added over a 20 minute period to a mixture of 9.6 g (0.25 mole) of sodium borohydride, 650 ml of tetrahydrofuran and 65 ml of methanol stirred at 0° under nitrogen, the temperature of the reaction mixture not being allowed to exceed 14°. The reaction mixture is stirred under nitrogen at 5°—10° for 30 minutes, and the tetrahydrofuran

and methanol are distilled at atmospheric pressure. 1 litre of toluene is added to the oily residue (200—300 ml) and the residual tetrahydrofuran is distilled at atmospheric pressure until the temperature reaches 108°—110°. The toluene solution is cooled to 40°, 1.3 litre of 0.5N sodium hydroxide is rapidly added, and the two phases are mixed and separated. The organic phase is heated to 50°—55°, 1.1 litre of *n*-hexane is added, the solution is cooled to 5°, and the precipitated colourless product is collected by filtration and vacuum dried for 16 hours at room temperature, m.p. 110°—111°.

c) 2-Chloromethyl-3-(4'-fluorophenyl)-1-methylindole (Reaction T; compound XXIIIa)

29.5 ml (0.404 mole) of thionyl chloride is added over a 10 minute period to a solution of 63.8 g (0.25 mole) of Compound XVIIIa in 650 ml of dry tetrahydrofuran (dried over molecular sieves) stirred at −7° under nitrogen. The reaction mixture is stirred at −5°—0° under nitrogen for 2.5 hours, 350 ml of toluene is added (with cooling to keep the temperature of the reaction mixture at or below 5°, tetrahydrofuran and excess thionyl chloride are distilled at 0.5—2 mm Hg and 0°—10° until the volume of the reaction mixture is about 400 ml, an additional 350 ml of toluene is added and another 100 ml of solvent is distilled at 0.5—1 mm Hg and 10°—20° to obtain a solution of the product in toluene.

d) 3-(4'-Fluorophenyl)-1-methyl-2-triphenylphosphonium-methylindole chloride (Reaction V; compound XXIVa)

A solution of 66.2 g (0.25 mole) of triphenylphosphine in 1 litre of toluene is added over a 3 minute period to the solution obtained in c), stirred at 15°—20° under nitrogen, and the reaction mixture is stirred at 108°—110° under nitrogen for 5 hours and cooled to 25°. The product is collected by filtration, washed twice with 50 ml portions of toluene and once with 50 ml of *n*-heptane and vacuum dried, m.p. 270°—271° (dec.).

e) (E)-4βR-(1',1'-dimethylethyl-diphenylsilyloxy)-6αS-[3'-(4''-fluorophenyl)-1'-methylindol-2'-yl-ethenyl]-2β-methoxy-3,4,5,6-tetrahydro-2H-pyran and the corresponding (Z) form (Reaction W; compounds XXVIa and XXVIb)

6.0 ml of 1.3M *n*-butyllithium/*n*-hexane (7.8 mmol) is added dropwise over a 7 minute period to a slurry of 4.0 g (7.47 mmol) of Compound XXIVa stripped from toluene at reduced pressure and dried under high vacuum prior to use in 100 ml of dry tetrahydrofuran (freshly distilled from sodium and benzophenone) stirred at room temperature under nitrogen. The reaction mixture is cooled to 0°, and 2.98 g (7.48 mmol) of compound

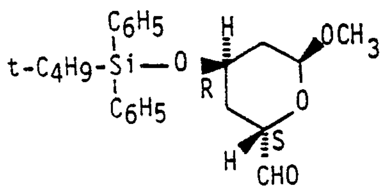

(stripped from toluene at reduced pressure and dried under high vacuum prior to use) in 20 ml of dry tetrahydrofuran is added dropwise over a 5 minute period, an additional 10 ml of dry tetrahydrofuran is added, and the reaction mixture is maintained at about 0° for 45 minutes, allowed to warm to room temperature and maintained at room temperature for 17 hours, the reaction mixture being stirred under nitrogen throughout. The reaction mixture is poured into 500 ml of water and extracted four times with 250 ml portions of diethyl ether. The diethyl ether extracts are combined and dried over anhydrous magnesium sulfate and then over anhydrous sodium sulfate and evaporated at reduced pressure. The last traces of diethyl ether are removed under high vacuum to obtain a semi-solid residue. The residue is subjected to medium pressure liquid chromatography utilizing a silica gel column and methylene chloride as the eluant, with those fractions containing one product and one or more contaminants or a mixture of the products (with or without one or more contaminants) as determined by thin layer chromatography being recycled, to obtain the (E) (*i.e., trans*) olefin (Compound XXVIa) as an orange foam and the (Z) (*i.e., cis*) olefin (Compound XXVIb) also as an orange foam.

f) (E)-4βR-(1',1'-dimethylethyl-diphenylsilyloxy)-6αS-[3'-(4''-fluorophenyl)-1'-methylindol-2'-yl-ethenyl]-2-hydroxy-3,4,5,6-tetrahydro-2H-pyran; compound XXVIIa and its 6βR isomer; compound XXVIIb (Reaction X)

1.18 g (1.9 mmol) of compound XXVIa is dissolved in 56 ml of glacial acetic acid, 37.2 ml of tetrahydrofuran is added, and 18.6 ml of distilled water is slowly added, the reaction mixture being stirred at room temperature throughout. The reaction mixture is stirred at 60° for 18.5 hours and allowed to cool. The tetrahydrofuran is evaporated at reduced pressure, and the reaction mixture is poured into 500 ml of distilled water and extracted four times with 300 ml portions of diethyl ether. The diethyl ether extracts are combined, washed with saturated sodium bicarbonate solution (until no gas is evolved upon shaking), dried over anhydrous magnesium sulfate and then over anhydrous sodium sulfate and evaporated to dryness at reduced pressure. The last traces of solvent are removed under high vacuum to obtain a yellow foam. Flash chromatography of the foam utilizing 250 g of silica gel and 1:1 (by volume) diethyl ether/*n*-hexane as the eluant yielded Compound XXVIIa and Compound XXVIIb.

g) (E)-4βR-(1′,1′-dimethylethyl-diphenylsilyloxy)-6αS-[3′-(4″-fluorophenyl)-1′-methylindol-2′-yl-ethenyl]-3,4,5,6-tetrahydro-2H-pyran-2-one (Reaction Y; compound XXVIIIa)

A solution of 236.8 mg (0.391 mmol) of compound XXVIIa in 8 ml of acetone (passed through a column of Activity I alumina immediately prior to use) is added to 137.5 mg (1.174 mmol) of N-methylmorpholine N-oxide (obtained by heating N-methylmorpholine N-oxide hydrate at 90° for 2—3 hours under high vacuum), the reaction mixture is stirred at room temperature under nitrogen until the solid dissolves, 23.5 mg (0.025 mmol) of dichlorotris(triphenylphosphine)ruthenium (II) is added, and the reaction mixture is stirred under nitrogen for 55 minutes 10 ml of diethyl ether is added, and the resulting solid is washed several times with diethyl ether. The diethyl ester washings are combined, the diethyl ether is evaporated at reduced pressure to near dryness, and the residue is dissolved in 100 ml of diethyl ether. The diethyl ether solution is washed twice with 100 ml portions of ice-cold 2.5% hydrochloric acid, twice with 100 ml portions of saturated sodium bicarbonate solution and once with 100 ml of saturated sodium chloride solution, dried over anhydrous sodium sulfate and evaporated to dryness at reduced pressure to obtain the crude product as a yellow oil.

h) (E)-*trans*-6S-[3′-(4″-fluorophenyl)-1′-methylindol-2′-ylethenyl]-4R-hydroxy-3,4,5,6-tetrahydro-2H-pyran-2-one (Process d)/deprotection; compound no. 17)

113 μl of glacial acetic acid is added dropwise to a solution of 237.5 mg (0.391 mmol) of crude Compound XXVIIIa in 18 ml of dry tetrahydrofuran stirred at room temperature under nitrogen followed by the dropwise addition of 1.564 ml of 1M tetra-*n*-butylammonium fluoride/tetrahydrofuran. The reaction mixture is stirred at room temperature under nitrogen for 2 hours, poured into 200 ml of ice-cold water and extracted four times with 75 ml portions of diethyl ether. The organic phases are combined, washed once with 300 ml of saturated sodium bicarbonate solution and once with 300 ml of saturated sodium chloride solution and dried over anhydrous sodium sulfate. The solvent is evaporated at reduced pressure, with the last traces being evaporated under high vacuum to obtain a yellow oil which is triturated with diethyl ether to obtain the product as a pale yellow solid. Additional product is obtained from the mother liquor by repeating this procedure three times, m.p. 139°—140°.

A second batch obtained by resoluton of the racemate had an $[\alpha]_D^{25} = -18.5°$ ($CHCl_3$, c = 0.2 g) [(cf. Example 3)b)ii)].

The corresponding (E), *cis*, 6R,4R isomer is obtained analogously (compound no. 18) starting from compound XXVIIb.

The corresponding (Z), *trans*, 6S,4R isomer is obtained analogously; compound no. 19. $[\alpha]_D^{25} = +136.935°$ ($CH_2Cl_2$, c = 1.24 g) starting from XXVIb.

Example 10

(E)-*Trans*-6S-[3′-(4″-fluorophenyl)-1′-(1″-methylethyl)indol-2′-ylethenyl]-4R-hydroxy-3,4,5,6-tetrahydro-2H-pyran-2-one (compound no. 20)

Obtained analogously to Example 9 starting from XXIIAa and the lactone illustrated is Example 9)e). $[\alpha]_D^{25} = -15.84°$ ($CHCl_3$, c = 1.3 g).

Compounds 1 to 20 have the following structures:

Compound no. 1: R = 4-F-$C_6H_4$-; Ro = $CH_3$; $R_2$ = $R_3$ = H; X =(E)CH=CH; Z = IIa; $R_6$ = H; $R_7$ = $CH_3$ (mixture of 4 stereoisomers).

Compound no. 2: R = 4-F-$C_6H_4$-; Ro = $CH_3$; $R_2$ = $R_3$ = H; X = (E)CH=CH; Z = IIa; $R_6$ = H; $R_7$ = K (mixture of 4 stereoisomers).

Compound no. 3: R = 4-F-$C_6H_4$-; Ro = $CH_3$; $R_2$ = $R_3$ = H; X = (E)CH=CH; Z = IIa; $R_6$ = $R_7$ = H (mixture of 4 stereoisomers).

Compound no. 4: R = 4-F-$C_6H_4$-; Ro = $CH_3$; $R_2$ = $R_3$ = H; X = (E)CH=CH; Z = IIb; $R_6$ = H (mixture of 4 stereoisomers).

Compound no. 5: R = 4-F-$C_6H_4$-; Ro = $CH_3$; $R_2$ = $R_3$ = H; X = (E)CH=CH; Z = IIb; $R_6$ = H (*trans* racemate).

Compound no. 6: R = 4-F-$C_6H_4$-; Ro = $CH_3$; $R_2$ = $R_3$ = H; X = (E)CH=CH; Z = IIb; $R_6$ = H (*cis* racemate).

Compound no. 7: R = 4-F-$C_6H_4$-; Ro = i-$C_3H_7$; $R_2$ = $R_3$ H; X = (E)CH=CH; Z = IIa; $R_6$ = H; $R_7$ = $CH_3$ (*erythro* racemate).

Compound no. 8: R = 4-F-$C_6H_4$-; Ro = i-$C_3H_7$; $R_2$ = $R_3$ = H; X = (E)CH=CH; Z = IIa; $R_6$ = $R_7$ = H (*erythro* racemate).

Compound no. 9 (= cmpd. no. 13): R = 4-F-$C_6H_4$-; Ro = i-$C_3H_7$; $R_2$ = $R_3$ = H; X = (E)CH=CH; Z = IIa; $R_6$ = H; $R_7$ = Na (*erythro* racemate).

Compound no. 10: R = 4-F-$C_6H_4$-; Ro = i-$C_3H_7$; $R_2$ = $R_3$ = H; X = (E)CH=CH; Z = IIb; $R_6$ = H (mixture of 4 stereoisomers).

Compound no. 11: R = 4-F-$C_6H_4$-; Ro = i-$C_3H_7$; $R_2$ = $R_3$ = H; X = (E)CH=CH; Z = IIb; $R_6$ = H (*trans* racemate).

Compound no. 12: R = 4-F-$C_6H_4$-; Ro = i-$C_3H_7$; $R_2$ = $R_3$ = H; X = (E)CH=CH; Z = IIb; $R_6$ = H (*cis* racemate).

Compound no. 13: (= cmpd. no. 9).

Compound no. 14: R = 4-F-$C_6H_4$-; Ro = i-$C_3H_7$; $R_2$ = $R_3$ = H; X = (E)CH=CH; Z = IIa; $R_6$ = H; $R_7$ = Na (*erythro*/3R,5S isomer).

Compound no. 15: R = 4-F-$C_6H_4$-; Ro = i-$C_3H_7$; $R_2$ = $R_3$ = H; X = (E)CH=CH; Z = IIa; $R_6$ = H; $R_7$ = Na (*erythro*/3S,5R isomer).

Compound no. 16: R = 4-F-$C_6H_4$-; Ro = i-$C_3H_7$; $R_2$ = $R_3$ = H; X = (E)CH=CH; Z = IIa; $R_6$ = H; $R_7$ = Na (*threo* racemate).

Compound no. 17: R = 4-F-$C_6H_4$-; Ro = $CH_3$; $R_2$ = $R_3$ = H; X = (E)CH=CH; Z = IIb; $R_6$ = H (*trans*/6S,4R isomer).

Compound no. 18: R = 4-F-$C_6H_4$-; Ro = $CH_3$; $R_2$ = $R_3$ = H; X = (E)CH=CH; Z = IIb; $R_6$ = H (*cis*/6R,4R isomer).

Compound no. 19: R = 4-F-$C_6H_4$-; Ro = $CH_3$; $R_2$ = $R_3$ = H; X = (Z)CH=CH; Z = IIb; $R_6$ = H (*trans*/6S,4R isomer).

Compound no. 20: R = 4-F-$C_6H_4$-; Ro = i-$C_3H_7$; $R_2$ = $R_3$ = H; X = (E)CH=CH; Z = IIb; $R_6$ = H (*trans*/6S,4R isomer).

The compounds of the following tables may be prepared analogously to the preceding examples or according to processes set out hereinbefore.

[Abbreviations: DB = direct bond
D = mixture of diastereoisomers (four stereoisomers)
E = *erythro* racemate (two stereoisomers)
T = *threo* racemate (two stereoisomers)]

TABLE I

(Compounds of type IAa: $R_6$ = H; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_7$ | Isomer(s) | m.p. |
|---|---|---|---|---|---|---|---|---|---|
| 21 | $CH_3$ | H | H | H | H | 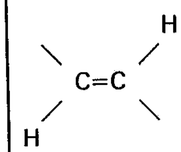 | $CH_3$ | D | |
| 22 | $CH_3$ | H | H | H | H | " | $C_2H_5$ | D | Viscous oil |
| 23 | $CH_3$ | H | H | H | H | " | Na | D | |
| 24 | $CH_3$ | H | H | H | H | " | H | D | |
| 25 | $CH_3$ | H | H | H | H | " | Na | E | Amorphous solid |
| 26 | $CH_3$ | H | H | H | H | " | Na | T | Amorphous solid |
| 27 | $CH_3$ | 6-$OCH_2C_6H_5$ | H | 4-F | H | " | $C_2H_5$ | D | Solid foam |
| 28 | $CH_3$ | 6-$OCH_2C_6H_5$ | H | 4-F | H | " | K | D | |
| 29 | $CH_3$ | 6-$OCH_2C_6H_5$ | H | 4-F | H | " | Na | D | Amorphous solid |
| 30 | $CH_3$ | 6-$OCH_2C_6H_5$ | H | 4-F | H | " | H | D | |
| 31 | $CH_3$ | H | H | 4-F | H | DB | $CH_3$ | D | |
| 32 | $CH_3$ | H | H | 4-F | H | DB | Na | D | |
| 33 | $CH_3$ | H | H | 4-F | H | DB | H | D | |

TABLE I cont.

(Compounds of type IAa: $R_6$ = H; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_7$ | Isomer(s) | m.p. |
|---|---|---|---|---|---|---|---|---|---|
| 34 | $CH_3$ | H | H | 3-$CH_3$ | 4-$CH_3$ | 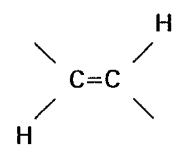 | $C_2H_5$ | D | Oil |
| 35 | $CH_3$ | H | H | 3-$CH_3$ | 4-$CH_3$ | " | K | D | |
| 36 | $CH_3$ | H | H | 3-$CH_3$ | 4-$CH_3$ | " | H | D | |
| 37 | *i*-$C_2H_7$ | H | H | 4-F | H | " | $C_2H_5$ | D | |
| 38 | " | H | H | 4-F | H | " | $CH_3$ | D | Viscous oil |
| 39 | " | H | H | 4-F | H | " | K | D | |
| 40 | " | H | H | 4-F | H | " | H | D | |
| 41 | $CH_3$ | 4-$OCH_2C_6H_5$ | H | 4-F | H | " | $C_2H_5$ | D | Viscous oil |
| 42 | $CH_3$ | 4-$OCH_2C_6H_5$ | H | 4-F | H | " | K | D | |
| 43 | $CH_3$ | 4-$OCH_2C_6H_5$ | H | 4-F | H | " | H | D | |
| 44 | $CH_3$ | H | H | 3-$CH_3$ | 5-$CH_3$ | " | $C_2H_5$ | D | Viscous oil |
| 45 | $CH_3$ | H | H | 3-$CH_3$ | 5-$CH_3$ | " | K | D | |
| 46 | $CH_3$ | H | H | 3-$CH_3$ | 5-$CH_3$ | " | H | D | |
| 47 | $CH_3$ | H | H | 3-$CH_3$ | 5-$CH_3$ | " | Na | E | Amorphous solid |

TABLE I cont.
(Compounds of type IAa: $R_6$ = H; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_7$ | Isomer(s) | m.p. |
|---|---|---|---|---|---|---|---|---|---|
| 48 | $CH_3$ | H | H | 3-$CH_3$ | 5-$CH_3$ | 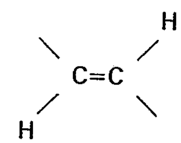 | Na | T | Amorphous solid |
| 49 | $CH_3$ | 5-Cl | H | 4-F | H | " | $C_2H_5$ | D | 96°—105° |
| 50 | $CH_3$ | 5-Cl | H | 4-F | H | " | K | D | |
| 51 | $CH_3$ | 5-Cl | H | 4-F | H | " | H | D | |
| 52 | $CH_3$ | 5-$OCH_3$ | H | 4-F | H | " | $C_2H_5$ | D | Viscous oil |
| 53 | $CH_3$ | H | H | 4-F | H | " | Na | E | 193°—196° (dec.) |
| 54 | $CH_3$ | 7-$OCH_2C_6H_5$ | H | 4-F | H | " | $C_2H_5$ | D | 78°—82° |
| 55 | $CH_3$ | 7-$OCH_2C_6H_5$ | H | 4-F | H | " | Na | E | Amorphous solid |
| 56 | $CH_3$ | 7-$OCH_2C_6H_5$ | H | 4-F | H | " | Na | T | Amorphous solid |
| 57 | $CH_3$ | 5-$OCH_2C_6H_5$ | H | 4-F | H | " | $C_2H_5$ | D | Viscous oil |
| 58 | $C_6H_5$-$CH_2CH_2$ | H | H | 4-F | H | " | $CH_3$ | D | Oil |
| 59 | $C_6H_5$-$CH_2CH_2$ | H | H | 4-F | H | " | Na | E | Amorphous solid |

TABLE I cont.

(Compounds of type IAa: $R_6$ = H; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_7$ | Isomer(s) | m.p. |
|---|---|---|---|---|---|---|---|---|---|
| 60 | $C_2H_5$ | H | H | 4-F | H | 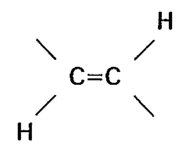 | $C_2H_5$ | D | Viscous oil |
| 61 | $C_2H_5$ | H | H | 4-F | H | " | Na | E | Amorphous solid |
| 62 | $C_2H_5$ | H | H | 4-F | H | " | Na | T | Amorphous solid |
| 63 | *i*-$C_3H_7$ | 4-$CH_3$ | 6-$CH_3$ | 4-F | H | " | $C_2H_5$ | D | 107°—110° |
| 64 | " | 4-$CH_3$ | 6-$CH_3$ | 4-F | H | " | Na | E | Solid foam |
| 65 | " | 4-$CH_3$ | 6-$CH_3$ | 4-F | H | " | Na | T | Solid foam |
| 66 | " | H | H | 3-$CH_3$ | 5-$CH_3$ | " | $CH_3$ | D | Viscous oil |
| 67 | " | H | H | 3-$CH_3$ | 5-$CH_3$ | " | Na | E | 183°—186° |
| 68 | " | 5-cyclohexyl | H | 4-F | H | " | $CH_3$ | D | Viscous oil |
| 69 | " | " | H | 4-F | H | " | Na | E | Solid foam |
| 70 | " | " | H | 4-F | H | " | Na | T | Solid foam |
| 71 | cyclohexyl | H | H | 4-F | H | " | $CH_3$ | D | Solid foam |

TABLE I cont.
(Compounds of type IAa: $R_5$ = H; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_7$ | Isomer(s) | m.p. |
|---|---|---|---|---|---|---|---|---|---|
| 72 | cyclohexyl | H | H | 4-F | H | 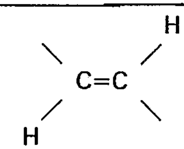 | Na | E | Amorphous solid |
| 73 | cyclohexyl | H | H | 4-F | H | " | Na | T | Amorphous solid |
| 74 | *i*-$C_3H_7$ | 6-$OCH_2C_6H_5$ | H | 4-F | H | " | $C_2H_5$ | D | Solid foam |
| 75 | " | H | H | 3-$CH_3$ | 4-F, 5-$CH_3$ | " | $C_2H_5$ | D | Viscous oil |
| 76 | *i*-$C_3H_7$ | H | H | 2-$CH_3$ | H | | Na | E | Amorphous solid |
| 77 | " | H | H | 2-$CH_3$ | H | " | Na | T | Amorphous solid |
| 78 | " | H | H | 3-$CH_3$ | 4-F | " | Na | E | Amorphous solid |
| 79 | " | H | H | 3-$CH_3$ | 4-F | " | Na | T | Amorphous solid |
| 80 | " | H | H | 3-$CH_3$ | 4-F, 5-$CH_3$ | " | Na | E | Amorphous solid |
| 81 | " | 6-$OCH_2C_6H_5$ | H | 4-F | H | " | Na | E | 180°—182° (dec.) |
| 138 | " | 4-i-$C_3H_7$ | 6-i$C_3H_7$ | 4-F | H | " | $C_2H_5$ | E | 123.5—125° |
| 140 | *i*-$C_4H_9$ | H | H | 4-F | H | " | Na | E | 140—160° (dec.) |

TABLE I cont.

(Compounds of type IAa: $R_6$ = H; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_7$ | Isomer(s) | m.p. |
|---|---|---|---|---|---|---|---|---|---|
| 142 | $i$-$C_3H_7$ | H | H | 2-$CH_3$ | 4-F | 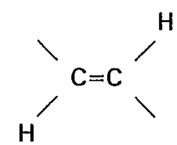 | Na | E | 155—168° (dec.) |
| 143 | " | H | H | 2-$CH_3$ | 4-F | " | $CH_3$ | D (mostly E) | |
| 144 | $i$-$C_4H_9$ | H | H | 4-F | H | " | $CH_3$ | D (mostly E) | |

TABLE II
(Compounds of type IAb: $R_6$ = H; when only one substituent or H appears in column "$R_5$, $R_{5a}$" then $R_{5a}$ = H)

| No. | $R_1$ | $R_2$ | $R_3$ | $R_4$ | t9m8Cmpd. $R_5$, $R_{5a}$ | X | Isomer(s) | m.p. |
|---|---|---|---|---|---|---|---|---|
| 82 | $CH_3$ | H | H | H | H | 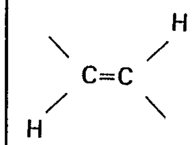 | *cis* | Solid foam |
| 83 | $CH_3$ | H | H | H | H | " | *trans* | 119.5—121° |
| 84 | $CH_3$ | 6-$OCH_2C_6H_5$ | H | 4-F | H | " | mixture: ~ 75% *trans* ~ 25% *cis* | 145—146° |
| 85 | $CH_3$ | H | H | 4-F | H | -$CH_2CH_2$- | *cis* | |
| 86 | $CH_3$ | H | H | 4-F | H | -$CH_2CH_2$- | *trans* | 164—169° (dec.) |
| 87 | $CH_3$ | H | H | 3-$CH_3$ | 4-$CH_3$ | 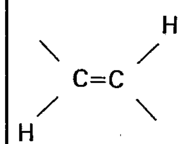 | *cis* | Solid foam |
| 88 | $CH_3$ | H | H | 3-$CH_3$ | 4-$CH_3$ | " | *trans* | Solid foam |
| 89 | *i*-$C_3H_7$ | 4-$CH_3$ | 6-$CH_3$ | 4-F | H | " | >95% *cis* | 150.5—151° |
| 90 | " | 4-$CH_3$ | 6-$CH_3$ | 4-F | H | " | *trans* | 146—147° |
| 91 | $CH_3$ | 4-$OCH_2C_6H_5$ | H | 4-F | H | " | *cis* | |
| 92 | $CH_3$ | 4-$OCH_2C_6H_5$ | H | 4-F | H | " | *trans* | |
| 93 | $CH_3$ | H | H | 3-$CH_3$ | 5-$CH_3$ | " | *cis* | Solid foam |
| 94 | $CH_3$ | H | H | 3-$CH_3$ | 5-$CH_3$ | " | *trans* | Solid foam |
| 95 | $CH_3$ | 5-Cl | H | 4-F | H | " | *cis* | 165.5—166° |

TABLE II cont.
(Compounds of type IAb: $R_6$ = H; when only one substituent or H appears in column "$R_5$, $R_{5a}$" then $R_{5a}$ = H)

| No. | $R_1$ | $R_2$ | $R_3$ | $R_4$ | t9m8Cmpd. $R_5$, $R_{5a}$ | X | Isomer(s) | m.p. |
|---|---|---|---|---|---|---|---|---|
| 96 | $CH_3$ | 5-Cl | H | 4-F | H | 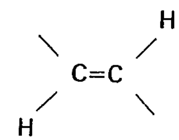 | *trans* | 157.5—159° |
| 97 | $CH_3$ | 5-$OCH_3$ | H | 4-F | H | " | *cis* | Solid foam |
| 98 | $CH_3$ | 5-$OCH_3$ | H | 4-F | H | " | *trans* | 102—105° |
| 99 | $CH_3$ | 7-$OCH_2C_6H_5$ | H | 4-F | H | " | *cis* | 127—128.5° |
| 100 | $CH_3$ | 7-$OCH_2C_6H_5$ | H | 4-F | H | " | *trans* | 140.5°—141.5° |
| 101 | $CH_3$ | 5-$OCH_2C_6H_5$ | H | 4-F | H | " | mixture: ~ 80% *cis* ~ 20% *trans* | 118—119° |
| 102 | $CH_3$ | " | H | 4-F | H | " | *trans* | 118—119° |
| 103 | $C_6H_5CH_2CH_2$ | H | H | 4-F | H | " | *cis* | Solid foam |
| 104 | " | H | H | 4-F | H | " | *trans* | Solid foam |
| 105 | *i*-$C_3H_7$ | H | H | 3-$CH_3$ | 5-$CH_3$ | " | *cis* | 108—110° |
| 106 | " | H | H | 3-$CH_3$ | 5-$CH_3$ | " | *trans* | 145—147° |
| 107 | $C_2H_5$ | H | H | 4-F | H | " | *cis* | 133.5—135° |
| 108 | " | H | H | 4-F | H | " | *trans* | 136—137° |
| 109 | *i*-$C_3H_7$ | H | H | 3-$CH_3$ | 4-F | " | *cis* | 120—123° |

TABLE II cont.
(Compounds of type IAb: $R_6$ = H; when only one substituent or H appears in column "$R_5$, $R_{5a}$" then $R_{5a}$ = H)

| No. | $R_1$ | $R_2$ | $R_3$ | $R_4$ | t9m8Cmpd. $R_5$, $R_{5a}$ | X | Isomer(s) | m.p. |
|---|---|---|---|---|---|---|---|---|
| 110 | *i*-$C_3H_7$ | H | H | 3-$CH_3$ | 4-F | 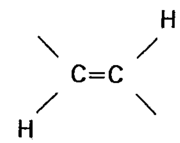 | *trans* | 140.5—141.5° |
| 111 | " | 5-cyclo-hexyl | H | 4-F | H | " | *cis* | Solid foam |
| 112 | " | " | H | 4-F | H | " | *trans* | 162—166° |
| 113 | cyclohexyl | H | H | 4-F | H | " | *cis* | Solid foam |
| 114 | " | H | H | 4-F | H | " | *trans* | Solid foam |
| 115 | *i*-$C_3H_7$ | H | H | 2-$CH_3$ | H | " | *cis* | Solid foam |
| 116 | " | H | H | 2-$CH_3$ | H | " | *trans* | Solid foam |
| 117 | " | H | H | 3-$CH_3$ | 4-F, 5-$CH_3$ | " | mixture: ~88% *cis* ~12% *trans* | 107—113° |
| 118 | " | H | H | 3-$CH_3$ | 4-F, 5-$CH_3$ | " | *trans* | 166.5—167.5° |
| 119 | " | 6-$OCH_2C_6H_5$ | H | 4-F | H | " | *trans* | 152—153° (dec.) |
| 139 | *i*-$C_4H_9$ | H | H | 4-F | H | " | *trans* | 51—55° (dec.) |
| 141 | *i*-$C_3H_7$ | H | H | 2-$CH_3$ | 4-F | " | *trans* | 64—69° |

TABLE III
(Compounds of type IBa; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_6$ | $R_7$ | Isomer(s) | m.p. |
|---|---|---|---|---|---|---|---|---|---|---|
| 120 | $CH_3$ | H | H | 4-F | H | 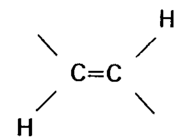 | H | $C_2H_5$ | D | |
| 121 | $CH_3$ | H | H | 4-F | H | " | H | K | D | |
| 122 | $CH_3$ | H | H | 4-F | H | " | H | H | D | |
| 123 | $C_2H_5$ | 5-$OCH_3$ | H | 3-$CH_3$ | H | -$CH_2CH_2$- | $CH_3$ | $CH_3$ | D | |
| 124 | $C_2H_5$ | 5-$OCH_3$ | H | 3-$CH_3$ | H | -$CH_2CH_2$- | $CH_3$ | K | D | |
| 125 | $C_2H_5$ | 5-$OCH_3$ | H | 3-$CH_3$ | H | -$CH_2CH_2$- | $CH_3$ | H | D | |
| 126 | *i*-$C_3H_7$ | H | H | 4-F | H | 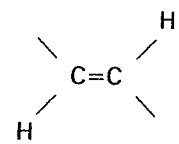 | H | $C_2H_5$ | D | |
| 127 | " | H | H | 4-F | H | " | H | K | D | |
| 128 | " | H | H | 4-F | H | " | H | H | D | |
| 129 | $CH_3$ | H | H | H | H | " | H | $C_2H_5$ | D | Viscous oil |

TABLE IV

(Compounds of type IBb; when only one substituent or H appears in column "$R_5$, $R_{5a}$" then $R_{5a}$ = H)

| Cmpd. No. | $R_1$ | $R_2$ | $R_3$ | $R_4$ | $R_5$, $R_{5a}$ | X | $R_6$ | Isomer(s) | m.p. |
|---|---|---|---|---|---|---|---|---|---|
| 130 | $CH_3$ | H | H | 4-F | H | 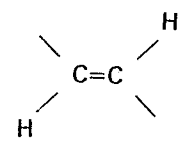 | H | *cis* | |
| 131 | $CH_3$ | H | H | 4-F | H | " | H | *trans* | |
| 132 | $C_2H_5$ | 5-$OCH_3$ | H | 3-$CH_3$ | H | $-CH_2CH_2-$ | $CH_3$ | *cis* | |
| 133 | $C_2H_5$ | 5-$OCH_3$ | H | 3-$CH_3$ | H | $-CH_2CH_2-$ | $CH_3$ | *trans* | |
| 134 | *i*-$C_3H_7$ | H | H | 4-F | H | 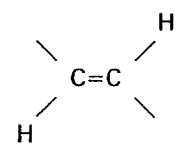 | H | *cis* | |
| 135 | " | H | H | 4-F | H | " | H | *trans* | |
| 136 | $CH_3$ | H | H | H | H | " | H | *cis* | Solid foam |
| 137 | $CH_3$ | H | H | H | H | " | H | *trans* | Viscous oil |

TABLE V

[Compounds of type IV (sub-type XX) wherein X is 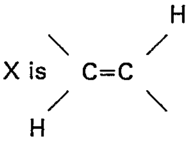 ]

| Cmpd. No. | R | $R_0$ | $R_2$ | $R_3$ | m.p. |
|---|---|---|---|---|---|
| IVe | 3,4-di-$CH_3$-$C_6H_3$- | $CH_3$ | H | H | 97—99° |
| IVf | 4-F-$C_6H_4$- | $C_6H_5CH_2CH_2$- | H | H | 86—88° |
| IVg | 4-F-$C_6H_4$- | *i*-$C_3H_7$ | 5-cyclohexyl | H | 162—167° |
| IVh | 2-$CH_3$-$C_6H_4$- | *i*-$C_3H_7$ | H | H | 190—193° |
| IVi | 3,5-di-$CH_3$-$C_6H_5$- | $CH_3$ | H | H | 117—118.5° |
| IVj | 4-F-$C_6H_4$- | $CH_3$ | 5-$OCH_3$ | H | 137—138.5° |
| IVk | 4-F-$C_6H_4$- | $CH_3$ | 6-$OCH_2C_6H_5$ | H | 128.5—131° |
| IVl | 4-F-$C_6H_4$- | $CH_3$ | 4-$OCH_2C_6H_5$ | H | 162.5—164° |
| IVm | 4-F-$C_6H_4$- | $CH_3$ | 5-Cl | H | 169.5—170.5° |
| IVn | $C_6H_5$- | $CH_3$ | H | H | 141.5—142.5° |
| IVo | 4-F-$C_6H_4$- | $CH_3$ | 7-$OCH_2C_6H_5$ | H | 140—141° |
| IVp | 4-F-$C_6H_4$- | $CH_3$ | 5-$OCH_2C_6H_5$ | H | 124.5—125° |
| IVq | 3-$CF_3C_6H_4$- | $CH_3$ | H | H | 124—124.5° |
| IVr | 4-F-$C_6H_4$- | $C_2H_5$ | H | H | 103—105° |
| IVs | 4-F-$C_6H_4$- | *i*-$C_3H_7$ | 4-$CH_3$ | 6-$CH_3$ | 189—190° |
| IVt | $C_6H_5$- | *i*-$C_3H_7$ | H | H | 111—112° |
| IVu | 3,5-di-$CH_3$-4-F-$C_6H_2$- | *i*-$C_3H_7$ | H | H | 114.5°—115° |
| IVv | 4-F-$C_6H_4$- | *i*-$C_3H_7$ | 6-$OCH_2C_6H_5$ | H | 118.5—120° |
| IVw | 4-F-$C_6H_4$- | *i*-$C_3H_7$ | 4-*i*-$C_3H_7$ | 6-*i*-$C_3H_7$ | 162—163° |
| IVx | $CH_3$ | $C_6H_5$- | H | H | crude amorphous solid |

TABLE VI
(Compounds of type XXIV)

| Compound No. | R | $R_0$ | $R_2$ | $R_3$ | $Y^{\ominus}$ | m.p. |
|---|---|---|---|---|---|---|
| XXIVb | 4-F-$C_6H_4$- | *i*-$C_3H_7$ | H | H | $Cl^{\ominus}$ | 236—239° |

Each of the compounds of Tables I and III denoted by a D in the Isomer(s) column is a mixture of four stereoisomers which may be separated. The four optically pure enantiomers that may be obtained may be designated as the 3R,5R, 3S,5S, 3R,5S and 3S,5R isomers. Except in the case of compounds 123—125 preferred are the 3R,5R and 3R,5S isomers and the racemate of which each is a constituent, *viz.,* the 3R,5R—3S,5S (*threo*) racemate and the 3R,5S—3S,5R (*erythro*) racemate, of which the latter is preferred. The preferred isomers of compounds 123—125 are the 3R,5R and 3R,5S isomers and the racemate of which is a constituent, *viz.,* the 3R,5R—3S,5S (*erythro*) racemate and the 3R,5S—3S,5R (*threo*) racemate, of which the former is preferred. Each of the compounds of Tables I and III denoted by an E in the Isomer(s) column is the *erythro* racemate which may be resolved to obtain the 3R,5S and 3S,5R enantiomers by, for example, (i) lactonization, (ii) conversion to a mixture of two diastereoisomeric silyloxy compounds, (iii) chromatographic separation of the diastereoisomeric silyloxy compounds, (iv) cleavage of the silyl group and (v) hydrolysis of the obtained optically pure lactone, as set forth in more detail above. Each of the compounds of Tables I and III denoted by a T in the Isomer(s) column is the *threo* racemate which may be resolved by, for example, the same procedure to obtain the 3R,5R and 3S,5S enantiomers.

Each of the compounds of Tables II and IV denoted by *cis* in the Isomer(s) column is the *cis* racemate and each of the compounds of these tables denoted by *trans* in the isomer column is the *trans* racemate, *cis* and *trans* referring to the relative positions of the hydrogen atoms in the 4- and 6-positions of the lactone ring. The *cis* racemates of compounds 85 and 132 may be resolved to obtain the 4R,6S and 4S,6R enantiomers and each of the other *cis* racemates of Tables II and IV may be resolved to obtain the 4R,6R and 4S,6S enantiomers, of which the 4R,6S and 4R,6R enantiomers are preferred. The *trans* racemates of Compounds 86 and 133 may be resolved to obtain the 4R,6R and 4S,6S enantiomers, and each of the other *trans* racemates of Tables II and IV may be resolved to obtain the 4R,6S and 4S,6R enantiomers, of which the 4R,6R and 4R,6S enantiomers are preferred. The *cis* and *trans* racemates may be resolved by Steps (ii)-(iv) of the procedure outlined in the preceding paragraph.

The following data were obtained for the preceding compounds. Unless otherwise stated the data are NMR spectra measured at 200 mHz. Shifts are in ppm, relative to tetramethylsilane.

*Abbreviations*:

s = singlet
d = doublet
dd = doublet of a doublet
t = triplet
q = quartet
Q = quintet
m = multiplet
br = broad
bs = broad singlet
dq = doublet of a quartet
dt = doublet of a triplet

Cmpd. no.

1 $CDCl_3$: 1.5—1.9 (m, 2H); 2.4—2.6 (m, 2H); 2.8—3.4 (br., 2H: $D_2O$ exch); 3.7 (s, 3H); 3.8 (s, 3H); 4.26 (m, 1H); 4.55 (m, 1H); 5.85—6.1 (m, 1H); 6.7 (2d, 1H); 7.05—7.55 (m, 8H).

4 $CDCl_3$: 1.6—3.0 (m, 5H); 3.82 (2s, 3H); 4.39 (m, 1H); 4.78 (m, $\frac{1}{2}$H): *cis*/$C_6$-H); 5.3 (m, $\frac{1}{2}$H:*trans*/$C_6$-H); 5.82—6.0 (m, 1H); 6.69—6.81 (2d, 1H); 7.05—7.6 (m, 8H).

11 $CDCl_3$: 1.68 (d, 6H); 1.75—2.05 (m, 3H); 2.55—2.82 (m, 2H); 4.38 (m, 1H); 4.82 (Q, 1H); 5.25 (m, 1H); 5.72 (q, 1H); 6.75 (d, 1H); 7.05—7.6 (m, 8H).

13 $CDCl_3/CD_3OD$: 1.55 (m, 1H); 1.6 (d, 6H), 2.2—2.45 (m 3H); 4.08 (m, 1H); 4.42 (m, 1H); 4.9 (Q, 1H); 5.75 (dd, 1H); 6.68 (d, 1H); 7.0—7.2 (m, 4H); 7.48—7.58 (m, 4H).

16 $D_2O$: 1.05 (d, 6h); 1.28 (m, 2H); 2.18 (d, 2H); 3.95 (m, 1H); 4.2 (m, 1H); 4.5 (m, 1H); 5.4 (dd, 1H); 6.4 (d, 1H); 6.5—7.2 (m, 8H).

18 $CDCl_3$: 1.71 (m, 1H); 2.05 (m, 1H); 2.31 (m, 1H); 2.52 (dd; $J_1$=17.5Hz; $J_2$=8Hz; 1H); 2.95 (dd, $J_1$=17.5Hz; $J_2$=5.5Hz; 1H); 3.85 (s, 3H); 4.31 (m, 1H); 4.81 (m, 1H); 5.97 (dd, $J_1$=16Hz; $J_2$=6Hz; 1H); 6.77 (d, J=16Hz; 1H); 7.09—7.72 (m, 8H).

19 $CDCl_3$: 0.75 (m, 1H); 1.14 (m, 1H); 1.45 (m, 1H); 2.48 (m, 2H); 3.76 (s, 3H); 4.1 (bs, 1H); 5.1 (m, 1H); 5.89 (dd, $J_1$=10.5Hz; $J_2$=10Hz; 1H); 6.7 (d, J=10.5Hz, 1H); 7.09—7.73 (m, 8H).

22 $CDCl_3$: 1.29 (t, 3H); 1.51—1.88 (m, 2H); 2.48 (d, 2H); 3.83 (s, 3H); 4.08—4.37 (m, 3H); 4.54 (m, 1H); 5.96 (m, 1H); 6.76 (m, 1H); 7—7.68 (m, 9H).

25 $D_2O$: 1.38—1.73 (m, 2H); 2.12—2.26 (m, 2H); 3.60 (s, 3H); 3.85 (m, 1H); 4.25 (m, 1H), 5.75 (dd, 1H); 6.52 (d, 1H); 6.96—7.50 (m, 9H).

26 $D_2O$: 1.41 (t, 2H); 2.20 (d, 2H); 3.36 (s, 3H); 4.0 (m, 1H); 4.2 (m, 1H); 5.66 (dd, 1H); 6.38 (d, 1H); 6.78—7.39 (m, 9H).

27 $CDCl_3$: 1.26 (t, 3H); 1.68 (m, 2H); 2.45 (d, 2H); 3.69 (s, 3H); 4.17 (q, 2H); 4.25 (m, 1H); 4.50 (m, 1H); 5.10 (s, 2H); 5.88 (m, 1H); 6.65 (m, 1H); 7.09 (t, 2H); 7.44 (m, 9H); 7.85 (d, 2H).

Cmpd. no.

29 $CDCl_3/CD_3OD$: 1.49—1.80 (m, 2H); 2.17—2.44 (m, 2H); 3.75 (s, 3H); 4.09 (m, 1H); 4.42 (m, 1H); 5.13 (s, 2H); 5.90 (dd, 1H); 6.63 (d, 1H); 6.77—6.92 (m, 2H); 7.09 (t, 2H); 7.27—7.54 (m, 8H).

34 $CDCl_3$: 1.28 (t, 3H); 1.6—1.8 (m, 2H); 2.3 (s, 6H); 2.48 (m, 2H); 3.82 (s, 3H); 4.18 (q, 2H); 4.3 (m, 1H); 4.55 (m, 1H); 6.0 (m, 1H); 6.75 (2d, 1H); 7.05—7.65 (m, 7H).

38 $CDCl_3$: 1.45—1.82 (m, 2H); 1.68 (d, 6H); 2.48 (m, 2H); 3.22 (d, 1H, exchangeable); 3.59 (m, 1H, exchangeable), 3.75 (s, 3H); 4.2 (m, 1H); 4.5 (m, 1H); 4.86 (q, 1H); 5.75 (m, 1H); 6.72 (2d, 1H); 7.05—7.55 (m, 8H).

41 $CDCl_3$: 1.28 (t, 3H); 1.61 (m, 2H); 2.42 (m, 2H); 3.80 (s, 3H); 4.13 (q, 2H); 4.45 (bs, 1H); 4.96 (s, 2H); 5.72 (m, 1H); 6.56 (m, 1H); 6.90 (m, 5H); 7.24 (m, 7H).

44 $CDCl_3$: 1.5—1.85 (m, 2H); 1.27 (t, 3H); 2.35 (s, 6H); 2.43—2.5 (m, 2H); 3.79 (s, 3H); 4.28 (m, 1H), 4.53 (m, 1H); 5.97 (m, 1H); 6.73 (m, 1H); 6.94—7.35 (m, 5H); 7.62 (d, 2H).

47 $D_2O$: 1.86 (s, 6H); 3.18 (s, 3H).

48 $D_2O$: 2.10 (s, 6H); 3.48 (s, 3H).

52 $CDCl_3$: 1.28 (t, 3H); 1.70 (m, 2H); 2.46 (d, 2H); 3.78 (s, 3H); 3.81 (s, 3H); 4.19 (q, 2H); 4.24 (bs, 1H); 5.89 (m, 1H); 6.68 (m, 1H); 6.94 (m, 2H); 7.17 (m, 3H); 7.41 (m, 2H).

55 $CDCl_3/CD_3OD$: 1.42—1.73 (m, 2H); 2.12—2.42 (m, 2H); 4.07 (s, 3H); 4.40 (m, 1H); 5.2 (s, 2H); 5.83 (dd, 1H); 6.53—6.75 (m, 2H); 6.89—7.14 (m, 3H); 7.27—7.50 (m, 8H).

56 $CDCl_3/CD_3OD$: 1.41—1.67 (m, 2H); 2.20—2.33 (m, 2H); 4.03 (s, 3H); 3.98—4.18 (m, 1H); 4.33 (m, 1H); 5.15 (s, 2H); 5.83 (dd, 1H); 6.59—6.73 (m, 2H); 6.85—7.13 (m, 4H); 7.24—7.50 (m, 6H).

57 $CDCl_3$: 1.28 (t, 3H); 1.51—1.87 (m, 2H); 2.37—2.62 (m, 2H); 3.75 (s, 3H); 4.08—4.33 (m, 3H); 4.52 (m, 1H); 5.02 (s, 2H); 5.90 (m, 1H); 6.57—6.73 (m, 1H); 6.92—7.47 (m, 12H).

58 $CDCl_3$: 1.4—1.8 (m, 2H); 2.45 (m, 2H); 2.84 (m, 1H, exchangeable); 3.1 (t, 2H); 3.45 (m, 1H, exchangeable); 3.72 (s, 3H); 4.2 (m, 1H); 4.42 (t, 2H); 5.72 (m, 1H); 6.4 (d, 1H); 7—7.55 (m, 13H).

59 $CDCl_3/CD_3OD$: 1.48 (m, 1H,); 2.25 (m, 3H); 2.92 (m, 2H); 4.2 (m, 4H); 5.75 (dd, 1H); 6.45 (d, 1H); 6.9—7.5 (m, 13H).

60 $CDCl_3$: 1.27 (t, 3H); 1.40 (t, 3H); ~ 1.40—1.85 (m, 2H); 2.33—2.61 (m, 2H); 4.03—4.35 (m, 5H); 4.53 (m, 1H); 5.89 (m, 1H); 6.60—6.78 (m, 1H); 7.0—7.57 (m, 8H).

61 $CDCl_3/CD_3OD$: 1.16—1.74 (m, 5H); 2.12—2.47 (m, 2H); 4.12 (m, 1H); 4.27 (q, 2H); 4.41 (m, 1H); 5.88 (dd, 1H); 6.65 (d, 1H); 7.0—7.56 (m, 8H).

62 $CDCl_3$: 1.40 (t, 3H); 1.50—1.68 (m, 2H); 2.19—2.38 (m, 2H); 4.12 (m, 1H); 4.28 (q, 2H); 4.47 (m, 1H); 5.90 (dd, 1H); 6.67 (d, 1H); 7.0—7.54 (m, 8H).

64 $CDCl_3/CD_3OD$: 1.15—1.6 (m, 2H); 1.65 (d, 6H); 2.01 (s, 3H); 2.1—2.4 (m, 2H); 2.45 (s, 3H); 3.92 (m, 1H); 4.29 (m, 1H); 4.84 (m, 1H); 5.58 (dd, 1H); 6.52 (d, 1H); 6.60 (s, 1H); 6.96—7.37 (m, 5H).

65 $CDCl_3/CD_3OD$: 1.3—1.65 (m, 2H); 1.67 (d, 6H); 2.01 (s, 3H); 2.15—2.35 (m, 2H); 2.43 (s, 3H); 3.98 (m, 1H); 4.34 (m, 1H); 4.85 (m, 1H); 5.54 (dd, 1H); 6.53 (d, 1H); 6.61 (s, 1H); 6.95—7.38 (m, 5H).

66 $CDCl_3$: 1.55 (m, 2H); 1.65 (d, 6H); 2.33 (s, 6H); 2.45 (m, 2H); 3.72 (s, 1H); 4.20 (m, 1H); 4.50 (m, 1H); 4.85 (Q, 1H); 5.75 (dd, 1H); 6.7 (dd, 1H); 6.9—7.6 (m, 7H).

68 $CDCl_3$: 1.1—1.6 (m, 7H); 1.62 (d, 6H); 1.62—1.94 (m, 5H); 2.4—2.62 (m, 3H); 3.0—3.6 (br, 2H); 3.75 (s, 3H); 4.15 (m, 1H); 4.5 (m, 1H); 4.82 (Q, 1H); 5.6—5.8 (m, 1H); 6.7 (d, 1H); 7.05—7.45 (m, 7H).

69 $CDCl_3/CD_3OD$: 1.15—1.6 (m, 7H); 1.65 (d, 6H); 1.7—1.95 (m, 5H); 2.15—2.44 (m, 2H); 2.48—2.62 (m, 1H); 4.04 (m, 1H); 4.38 (m, 1H); 4.85 (Q, 1H); 5.7 (dd, 1H); 6.65 (d, 1H); 7.0—7.15 (m, 3H); 7.3—7.5 (m, 4H).

70 $CDCl_3/CD_3OD$: 1.15—1.6 (m, 8H); 1.64 (d, 6H); 1.7—2 (m, 4H); 2.3 (m, 2H); 2.55 (m, 1H); 4.1 (m, 1H); 4.46 (m, 1H); 4.85 (Q, 1H); 5.72 (dd, 1H); 6.65 (d, 1H); 7.1 (m, 3H); 7.4 (m, 4H).

71 $CDCl_3$: 1.3—2.05 (m, 11H); 2.2—2.5 (m, 3H); 3.1—3.6 (br, 2H); 3.72 (s, 3H); 4.05—4.6 (m, 3H); 5.7 (m, 1H); 6.7 (m, 1H); 7.0—7.6 (m, 8H).

72 $CDCl_3/CD_3OD$: 1.5 (m, 4H); 1.95 (m, 6H); 2.3 (m, 4H); 4.1 (m, 1H); 5.72 (dd, 1H); 6.7 (d, 1H); 7.1 (m, 4H); 7.5 (m, 4H).

73 $CDCl_3/CD_3OD$: 1.2—1.62 (m, 5H); 1.74—2.05 (m, 5H); 2.13—2.46 (m, 4H); 4.14 (m, 1H); 4.46 (m, 1H); 5.75 (dd, 1H); 6.68 (d, 1H); 7.0—7.2 (m, 4H); 7.3—7.6 (m, 4H).

74 $CDCl_3$: 1.3 (t, 3H); 1.4—1.82 (m, 2H); 1.60 (d, 6H); 2.38—2.58 (m, 2H); 4.20 (q, 2H); 4.50 (m, 1H); 4.78 (m, 1H); 5.13 (s, 2H); 5.55—5.78 (m, 1H); 6.57—6.74 (m, 2H); 6.83 (d, 1H); 6.98—7.14 (m, 3H); 7.24—7.55 (m, 8H).

75 $CDCl_3$: 1.28 (t, 3H); 1.41—1.81 (m, 2H); 1.64 (d, 6H); 2.25—3.58 (m, 2H); 2.27 (s, 6H); 4.18 (q, 2H); 4.52 (m, 1H); 4.83 (m, 1H); 5.62—5.84 (m, 1H); 6.61—6.76 (m, 1H); 6.98—7.23 (m, 4H); 7.43—7.56 (m, 2H).

76 $CDCl_3/CD_3OD$: 1.3 (m, 1H); 1.5 (m, 1H); 1.7 (d, 6H); 2.1 (d, 3H); 2.22 (m, 2H); 3.9 (m, 1H); 4.3 (m, 1H); 4.9 (Q, 1H); 5.55 (dt, 1H); 6.65 (d, 1H); 6.95—7.55 (m, 8H).

77 $CDCl_3/CD_3OD$: 1.3—1.6 (m, 2H); 1.7 (d, 6H); 2.1 (d, 3H); 2.24 (m, 2H); 3.96 (m, 1H); 4.35 (m, 1H); 4.9 (Q, 1H); 5.58 (dd, 1H); 6.68 (d, 1H); 7.0—7.5 (m, 8H).

78 $CDCl_3/CD_3OD$: 1.45—1.81 (m, 2H); 1.57 (d, 6H); 2.15—2.46 (m, 2H); 2.30 (s, 3H); 4.88 (m, 1H); 5.73 (dd, 1H); 6.68 (d, 1H); 6.95—7.70 (m, 7H).

79 $CDCl_3/CD_3OD$: 1.42—1.62 (m, 2H); 1.68 (d, 6H); 2.17—2.35 (m, 5H); 4.1 (m, 1H); 4.45 (m, 1H); 4.90 (m, 1H); 5.78 (dd, 1H); 6.70 (d, 1H); 6.95—7.58 (m, 7H).

Cmpd. no.

80 $CDCl_3/CD_3OD$: 1.4—1.65 (m, 2H); 1.67 (d, 6H); 2.28 (d, 6H); 2.12—2.45 (m, 2H); 4.43 (m, 1H); 4.88 (m, 1H); 5.74 (dd, 1H); 6.67 (d, 1H); 6.98—7.22 (m, 4H); 7.53 (d, 2H).

82 $CDCl_3$: 1.61—3.0 (m, 5H); 3.87 (s, 3H); 4.29 (m, 1H); 4.79 (m, 1H); 5.98 (dd, 1H); 6.80 (d, 1H); 7.13 (t, 1H); 7.24—7.5 (m, 7H); 7.62 (d, 1H).

87 $CDCl_3$: 1.58 (s, 1H); 1.82 (m, 1H); 1.9 (m, 1H); 2.35 (s, 6H); 2.5 (q, 1H); 2.95 (q, 1H); 3.85 (s, 3H); 4.3 (m, 1H); 4.8 (m, 1H); 6.0 (q, 1H); 6.8 (d, 1H); 7.05—7.65 (m, 7H).

88 $CDCl_3$: 1.6 (s, 1H); 1.8—2.1 (m, 2H); 2.35 (s, 6H); 2.7 (m, 2H); 3.85 (s, 3H); 4.38 (m, 1H); 5.3 (m, 1H); 6.0 (q, 1H); 6.8 (d, 1H); 7.05—7.65 (m, 7H).

93 $CDCl_3$: 1.65—2.1 (m, 2H); 2.52—2.8 (m, 2H); 2.38 (s, 6H); 3.85 (s, 3H); 4.80 (m, 1H); 5.98 (m, 1H); 6.79 (d, 1H); 6.93—7.38 (m, 6H); 7.62 (d, 1H).

94 $CDCl_3$: 1.3—3.0 (m, 4H); 2.38 (s, 6H); 3.82 (s, 3H); 5.30 (m, 1H); 5.95 (dd, 1H); 6.78 (d, 1H); 6.93—7.40 (m, 6H); 7.62 (d, 1H).

97 $CDCl_3$: 1.61—3.02 (m, 5H); 3.83 (s, 3H); 4.31 (m, 1H); 4.78 (m, 1H); 5.90 (dd, 1H); 6.73 (d, 1H); 6.94 (m, 2H); 7.1—7.38 (m, 3H); 7.40 (m, 2H).

103 $CDCl_3$: 1.55 (m, 1H); 2.18 (m, 1H); 2.28 (s, 1H); 2.46 (1, 1H); 2.9 (q, 1H); 3.06 (t, 2H); 4.22 (m, 1H); 4.4 (t, 2H); 4.6 (m, 1H); 5.68 (dd, 1H); 6.4 (d, 1H); 7.05—7.55 (m, 13H).

104 $CDCl_3$: 1.55—1.95 (m, 2H); 2.42 (s, 1H, exchangeable); 2.65 (m, 2H); 3.05 (t, 2H); 4.3 (m, 1H); 4.4 (t, 2H); 5.15 (m, 1H); 5.68 (dd, 1H); 6.4 (d, 1H); 7.05—7.55 (m, 13H).

111 $CDCl_3$: 1.1—1.35 (m, 5H); 1.65 (d, 6H); 1.65—2.0 (m, 5H); 2.05—2.3 (m, 2H); 2.4—2.6 (m, 2H); 2.92 (dd, 1H); 4.25 (m, 1H); 4.78 (m, 2H); 5.65 (dd, 1H); 6.72 (d, 1H); 7.02—7.48 (m, 7H).

113 $CDCl_3$: 1.2—2.55 (m, 14H); 2.92 (q, 1H); 4.3 (m, 2H); 4.78 (m, 1H); 5.68 (dd, 1H); 6.75 (d, 1H); 7.0—7.6 (m, 8H).

114 $CDCl_3$: 1.2—2.05 (m, 10H); 2.2—2.45 (m, 2H); 2.7 (m, 2H); 4.3 (m, 2H); 5.25 (m, 1H); 5 65 (dd, 1H); 6.72 (d, 1H); 7.0—7.6 (m, 8H).

115 $CDCl_3$: 1.3—1.52 (m, 2H); 1.7 (d, 6H); 1.8 (m, 1H, exchangeable); 2.1 (d, 3H); 2.48 (dd, 1H); 2.9 (dd, 1H); 4.2 (m , 1H); 4.68 (m, 1H); 4.82 (Q, 1H); 5.5 (m, 1H); 6.7 (d, 1H); 7.0—7.5 (m, 8H).

116 $CDCl_3$: 1.68—1.9 (m, 9H); 2.1 (s, 3H); 2.64 (dq, 2H); 4.18 (m, 1H); 4.85 (Q, 1H); 5.16 (m, 1H); 5.45 (dt, 1H); 6.7 (d, 1H); 7.0—7.5 (m, 8H).

129 $CDCl_3$: 1.28 (t, 3H); 1.50—1.83 (m, 2H); 2.45 (d, 2H); 4.11—4.32 (m, 3H); 4.47 (m, 1H); 5.75 (m, 1H); 6.52 (m, 1H); 7.02—7.62 (m, 9H).

136 $CDCl_3$: 1.55—2.97 (m, 5H); 2.48 (s, 3H); 4.24 (m, 1H); 4.70 (s, 1H); 5.72 (dd, 1H); 6.57 (d, 1H); 7.04—7.63 (m, 9H).

137 $CDCl_3$: 1.18—2.08 (m, 3H); 2.5 (s, 3H); 2.53—2.82 (m, 2H); 4.38 (m, 1H); 5.21 (m, 1H); 5.72 (m, 1H); 6.58 (d, 1H); 7.07—7.65 (m, 9H).

143 $CDCl_3$: 1.42 (m, 2H); 1.66 (d, 6H); 2.09 (d, 3H); 2.42 (d, 2H); 3.72 (s, 3H); 4.05 (m, 1H); 4.39 (m, 1H); 4.84 (Q, 1H); 5.48 (dd, 1H); 6.67 (d, 1H); 6.90—7.56 (m, 7H).

144 $CDCl_3$: 0.95 (d, 6H); 1.57 (m, 2H); 2.25 (Q, 1H); 2.48 (d, 2H); 3.74 (s, 3H); 4.01 (d, 2H); 4.22 (m, 1H); 4.48 (m, 1H); 5.78 (dd, 1H); 6.67 (d, 1H); 7.04—7.51 (m, 8H).

**Claims**

1. Compounds of formula I

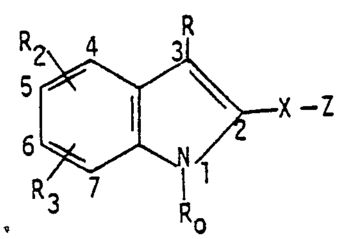

(I)

wherein one of R and $R_o$ is

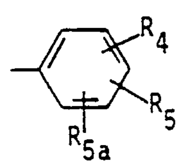

and the other is primary or secondary $C_{1-6}$alkyl, $C_{3-6}$cycloalkyl or phenyl-$(CH_2)_m$—, wherein

$R_4$ is hydrogen, $C_{1-4}$alkyl, $C_{1-4}$alkoxy (except t-butoxy), trifluoromethyl, fluoro, chloro, phenoxy or benzyloxy,

$R_5$ is hydrogen, $C_{1-3}$alkyl, $C_{1-3}$alkoxy, trifluoromethyl, fluoro, chloro, phenoxy or benzyloxy,

$R_{5a}$ is hydrogen, $C_{1-2}$alkyl, $C_{1-2}$alkoxy, fluoro or chloro, and

m is 1, 2 or 3,

with the provisos that both $R_5$ and $R_{5a}$ must be hydrogen when $R_4$ is hydrogen, $R_{5a}$ must be hydrogen when $R_5$ is hydrogen, not more than one of $R_4$ and $R_5$ is trifluoromethyl, not more than one of $R_4$ and $R_5$ is phenoxy and not more than one of $R_4$ and $R_5$ is benzyloxy,

$R_2$ is hydrogen, $C_{1-4}$alkyl, $C_{3-6}$cycloalkyl, $C_{1-4}$alkoxy, (except t-butoxy), trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos that $R_3$ must be hydrogen when $R_2$ is hydrogen, not more than one of $R_2$ and $R_3$ is trifluoromethyl, not more than one of $R_2$ and $R_3$ is phenoxy, and not more than one of $R_2$ and $R_3$ is benzyloxy,

X is $-(CH_2)_n-$ or $-CH=CH-$ (n=0, 1, 2 or 3), and

Z is

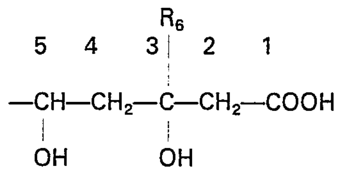

II

wherein $R_6$ is hydrogen or $C_{1-3}$alkyl in free acid form or in the form of a physiologically-hydrolysable and -acceptable ester of a δ-lactone thereof or in salt form.

2. A compound according to Claim 1, wherein one of R and $R_o$ is

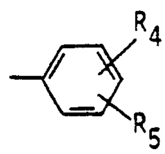

and the other is $C_{1-3}$alkyl, n-butyl or i-butyl,

wherein

$R_4$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy, and

$R_5$ is hydrogen, $C_{1-3}$alkyl, $C_{1-3}$alkoxy, trifluoromethyl, fluoro, chloro, phenoxy or benzyloxy, with the provisos that not more than one of $R_4$ and $R_5$ is trifluoromethyl, not more than one of $R_4$ and $R_5$ is phenoxy, and not more than one of $R_4$ and $R_5$ is benzyloxy,

$R_2$ is hydrogen, $C_{1-3}$alkyl, n-butyl, i-butyl, $C_{1-3}$alkoxy, n-butoxy, i-butoxy, trifluoromethyl, fluoro, chloro, phenoxy or benzyloxy,

$R_3$ is hydrogen, $C_{1-3}$alkyl, $C_{1-3}$alkoxy, trifluoromethyl, fluoro, chloro, phenoxy or benzyloxy, with the provisos tht not more than one of $R_2$ and $R_3$ is trifluoromethyl, not more than one of $R_3$ is trifluoromethyl, not more than one of $R_2$ and $R_3$ is phenoxy, and not more than one of $R_2$ and $R_3$ is benzyloxy,

X is $-(CH_2)_n-$ or

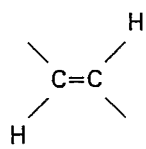

,

wherein n is 0, 1, 2 or 3, and

Z is 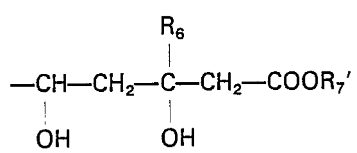 or 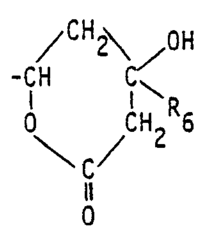

wherein

$R_6$ is hydrogen or $C_{1-3}$alkyl, and

$R_7'$ is hydrogen, $C_{1-3}$alkyl, n-butyl, i-butyl, t-butyl, benzyl or M, wherein M is a pharmaceutically acceptable cation.

3. A compound according to Claim 1 in free acid, salt or ester form.

4. A compound according to Claim 1 wherein the substituent meanings are as follows:

a) Ro is primary or secondary $C_{1-6}$alkyl not containing an asymmetric carbon atom,
R is

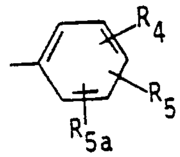

$R_2$ is hydrogen, $C_{1-3}$alkyl, $C_{1-3}$alkoxy, trifluoromethyl, fluoro, chloro, phenoxy or benzyloxy;
$R_3$ is hydrogen, $C_{1-3}$alkyl, $C_{1-2}$alkoxy, fluoro or chloro;
$R_4$ is hydrogen, $C_{1-3}$alkyl, $C_{1-3}$alkoxy, trifluoromethyl, fluoro, chloro, phenoxy or benzyloxy;
$R_5$ is hydrogen, $C_{1-2}$alkyl, $C_{1-2}$alkoxy, fluoro or chloro;
$R_{5a}$ is hydrogen or methyl;
X is $(CH_2)_m$, (wherein m is 1, 2 or 3) or (E)—CH=CH; and

Z is 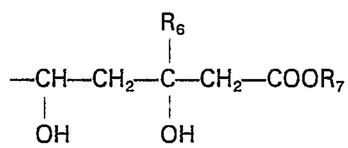

wherein
$R_6$ is hydrogen or $C_{1-2}$alkyl and
$R_7$ is hydrogen or $C_{1-3}$alkkyl;

b) Ro is $C_{1-3}$alkyl;
R is

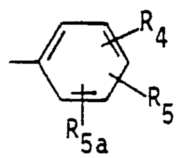

$R_2$ is hydrogen, $C_{1-3}$alkyl, methoxy, fluoro or chloro or 4-, 5- or 6-benzyloxy;
$R_3$ is hydrogen or $C_{1-3}$alkyl;
$R_4$ and $R_5$ are independently hydrogen, methyl, methoxy, fluoro or chloro;
$R_{5a}$ is hydrogen or methyl;
X is $(CH_2)_2$ or (E)—CH=CH; and

Z is 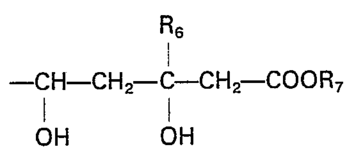

wherein
$R_6$ is hydrogen or methyl and
$R_7$ is hydrogen or $C_{1-2}$alkyl;

c) as b) except that $R_6$ is hydrogen;

d) Ro is $C_{1-3}$alkyl,
R is

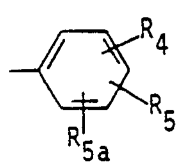

$R_2$ is hydrogen, $C_{1-3}$alkyl or 4- or 6-benzyloxy;
$R_3$ is hydrogen or methyl,
$R_4$ is hydrogen, methyl or fluoro,
$R_5$ is hydrogen or methyl,
$R_{5a}$ is hydrogen,
X is (E)—CH=CH and

Z is $-\underset{OH}{CH}-CH_2-\underset{OH}{CH}-CH_2-COOR_7'$

wherein $R_7$ is hydrogen or $C_{1-2}$alkyl.

5. A compound according to Claim 4 which when in salt form is in the form of its sodium, potassium or ammonium salt.

6. A compound according to any one of Claims 1 to 5 which when, Z is in other than lactone form is in erythro form.

7. A compound according to Claim 1, wherein R is p-fluoro-phenyl, Ro is isopropyl, $R_2$ and $R_3$ are hydrogen, X is (E)CH=CH, $R_6$ is hydrogen in *erythro* form.

8. A compound according to Claim 7 in free acid, salt or ester form.

9. A compound according to Claim 8 in free acid, sodium salt or methyl ester form.

10. Sodium *erythro*-(±)-(E)-3,5-dihydroxy-7[3'-(4''-fluorophenyl)-1'-(1''-methylethyl)-indol-2'-yl]hept-6-enoate.

11. Sodium *erythro*-(±)-(E)-3,5-dihydroxy-7[3'-(3'',5''-dimethylphenyl)-1'-(1''-methylethyl)-indol-2'-yl]-hept-6-enoate.

12. A compound according to Claim 7 in 3R,5S configuration and in the form of its sodium salt.

13. A pharmaceutical composition comprising a compound according to Claim 1 in free acid form or in the form of a physiologically-hydrolysable and -acceptable ester or a lactone thereof or in pharmaceutically acceptable salt form, together with a pharmaceutically acceptable diluent or carrier.

14. A compound according to Claim 1 in free acid form or in the form of physiologically-hydrolysable and -acceptable ester or a lactone thereof or in pharmaceutically acceptable salt form for use as a pharmaceutical.

15. A compound according to Claim 1 in free acid form or in the form of a physiologically-hydrolysable and -acceptable ester or a lactone thereof or in pharmaceutically acceptable salt form for use in inhibiting cholesterol biosynthesis or treating atherosclerosis.

16. A process for preparing a compound according to Claim 1 which comprises

a) when $R_6$ is hydrogen, reducing a compound of formula V

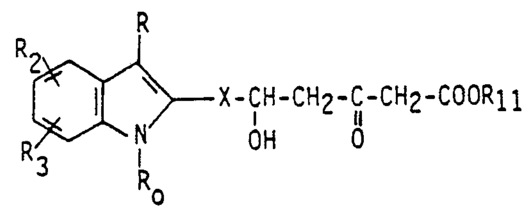

V

wherein $R_{11}$ is a radical forming a physiologically-hydrolysable and -acceptable ester and X, R, $R_o$, $R_2$ and $R_3$ are as defined above,

b) when $R_6$ = $C_{1-3}$alkyl, hydrolysing a compound of formula XII

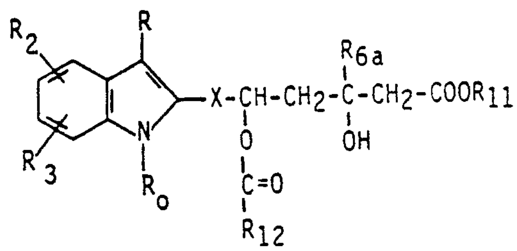

XII

wherein $R_{6a}$ is $C_{1-3}$alkyl, $R_{12}$ is an ester forming group and X, R, $R_o$, $R_3$ and $R_{11}$ are as defined above,

c) when X is —CH=CH— deprotecting a compound of formula XXVIII

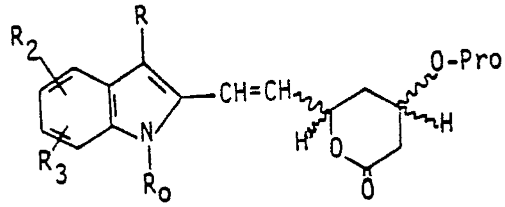

XXVIII

wherein Pro is a protecting group and R, $R_o$, $R_2$ and $R_3$ are as defined above,

d) hydrolysing a compound of formula I in the form of a physiologically-hydrolysable ester or a lactone or

e) esterifying or lactonising a compound of formula I in free acid form, and when a free carboxyl group is present, recovering the compound obtained in free acid form or in the form of a salt.

17. A compound of formula XX,

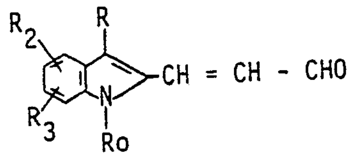

XX ·

wherein R, Ro, $R_2$ and $R_3$ have the meanings given in any one of claims 1 to 5.

**Patentansprüche**

1. Verbindung der Formel I

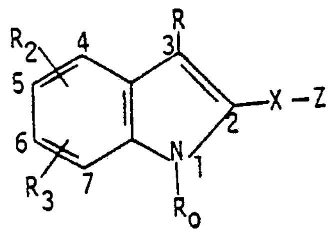

(I)

worin eines von R und Ro

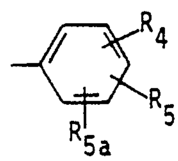

und das andere primäres oder sekundäres $C_{1-6}$Alkyl, $C_{3-6}$Cycloalkyl oder Phenyl-$(CH_2)_m$— ist worin

$R_4$ Wasserstoff, $C_{1-4}$Alkyl, $C_{1-4}$Alkoxy (ausgenommen t-Butyl), Trifluormethyl, Fluor, Chlor, Phenoxy oder Benzyloxy ist,

$R_5$ Wasserstoff, $C_{1-3}$Alkyl, $C_{1-3}$Alkoxy, Trifluormethyl, Fluor, Chlor, Phenoxy oder Benzyloxy ist,

$R_{5a}$ Wasserstoff, $C_{1-2}$Alkyl, $C_{1-2}$Alkoxy, Fluor oder Chlor ist, und

m 1, 2 oder 3 ist,

mit den Massgaben, dass sowohl $R_5$ als auch $R_{5a}$ Wasserstoff sein müssen wenn $R_4$ Wasserstoff ist, $R_{5a}$ Wasserstoff sein muss wenn $R_5$ Wasserstoff ist, nicht mehr als einer von $R_4$ und $R_5$ Trifluormethyl ist, nicht mehr als einer von $R_4$ und $R_5$ phenoxy ist und nicht mehr als einer von $R_4$ and $R_5$ benzyloxy ist,

$R_2$ Wasserstoff, $C_{1-4}$Alkyl, $C_{3-6}$Cycloalkyl, $C_{1-4}$Alkoxy, (ausgenommen t-Butoxy), Trifluormethyl, Fluor, Chlor, Phenoxy oder Benzyloxy ist,

$R_3$ Wasserstoff $C_{1-3}$Alkyl, $C_{1-3}$Alkoxy, Trifluormethyl, Fluor, Chlor, Phenoxy oder Benzyloxy,

mit den Massgaben dass $R_3$ Wasserstoff sein muss wenn $R_2$ Wasserstoff ist, nicht mehr als einer von $R_2$ und $R_3$ Trifluormethyl ist, nicht mehr als einer von $R_2$ und $R_3$ Phenoxy ist und nicht mehr als einer von $R_2$ und $R_3$ Benzyloxy ist,

X $—(CH_2)_n—$ oder —CH=CH— ist (n = 0, 1, 2 oder 3), und

$$Z \quad \overset{5}{—\underset{\displaystyle OH}{CH}}—\overset{4}{CH_2}—\overset{\displaystyle R_6}{\underset{\displaystyle OH}{\overset{3}{C}}}—\overset{2}{CH_2}—\overset{1}{COOH} \qquad II$$

ist worin $R_6$ Wasserstoff oder $C_{1-3}$Alkyl ist,

in Form der freien Säure oder in Form eines physiologisch-hydrolysierbaren und akzeptablen Esters oder ein δ-Lacton davon oder in Salzform.

2. Verbndung gemäss Anspruch 1 worin eines von R und Ro und das andere $C_{1-3}$Alkyl, n-Butyl oder i-Butyl ist, worin

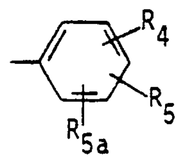

$R_4$ Wasserstoff, $C_{1-3}$Alkyl, n-Butyl, i-Butyl, $C_{1-3}$Alkoxy, n-Butoxy, i-Butoxy, Trifluormethyl, Fluor, Chlor, Phenoxy or Benzyloxy ist, und $R_5$ Wasserstoff, $C_{1-3}$Alkyl, $C_{1-3}$Alkoxy, Trifluormethyl, Fluor, Chlor, Phenoxy or Benzyloxy, mit den Massgaben, dass nicht mehr als einer von $R_4$ und $R_5$ Trifluormethyl ist, nicht mehr als einer von $R_4$ und $R_5$ Phenoxy ist und nicht mehr als einer von $R_4$ und $R_5$ Benzyloxy ist, $R_2$ Wasserstoff, $C_{1-3}$Alkyl, n-butyl, i-butyl, $C_{1-3}$Alkoxy, n-Butoxy, i-Butoxy, Trifluormethyl, Fluor, Chlor, Phenoxy or Benzyloxy ist, $R_3$ Wasserstoff, $C_{1-3}$Alkyl, $C_{1-3}$Alkoxy, Trifluormethyl, Fluor, Chlor, Phenoxy or Benzyloxy ist, mit den Massgaben, dass nicht mehr als einer von $R_2$ und $R_3$ Trifluormethyl ist, nicht mehr als einer von $R_2$ und $R_3$ Phenoxy ist und nicht mehr als einer von $R_2$ und $R_3$ Benzyloxy ist, X $-(CH_2)_n-$ oder

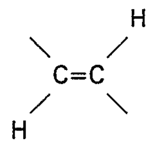 , ist

worin

n 0, 1, 2 oder 3 ist, und

Z 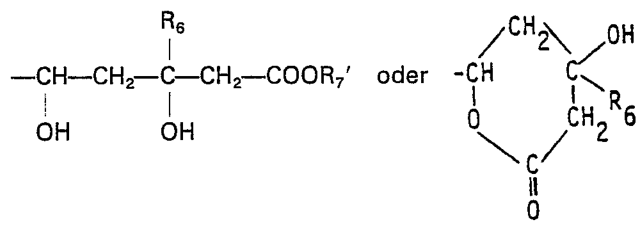 ist,

worin

$R_6$ Wasserstoff oder $C_{1-3}$Alkyl ist und $R_7'$ Wasserstoff, $C_{1-3}$Alkyl, n-Butyl, i-Butyl, t-Butyl, Benzyl oder M ist worin M ein pharmazeutisch akzeptables Kation ist.

3. Verbindung gemäss Anspruch 1 in Form der freien Säure oder in Salz- oder Esterform.

4. Verbindung gemäss Anspruch 1, worin die Substituenten-Bedeutungen wie folgt sind

a) Ro ist primäres oder sekondäres $C_{1-6}$Alkyl das kein asymmestrisches Kohlenstoffatom beinhaltet,

R ist

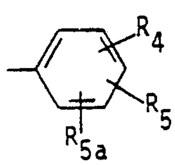

$R_2$ ist Wasserstoff, $C_{1-3}$Alkyl, $C_{1-3}$Alkoxy, Trifluormethyl, Fluor, Chlor, Phenoxy oder Benzyloxy;
$R_3$ ist Wasserstoff, $C_{1-3}$Alkyl, $C_{1-2}$Alkoxy, Fluor or Chlor;
$R_4$ ist Wasserstoff, $C_{1-3}$Alkyl, $C_{1-3}$Alkoxy, Triluormethyl, Fluor, Chlor, Phenoxy oder Benzyloxy;
$R_5$ ist Wasserstoff, $C_{1-2}$Alkyl, $C_{1-2}$Alkoxy, Fluor oder Chlor;
$R_{5a}$ ist Wasserstoff oder Methyl;
X ist $(CH_2)_m$, (worin m 1, 2 oder 3 ist) oder (E)—CH=CH; und

Z 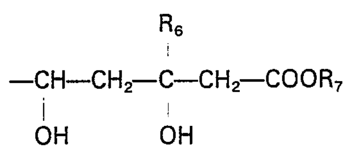 ist

worin

$R_6$ Wasserstoff oder $C_{1-2}$Alkyl ist und
$R_7$ Wasserstoff oder $C_{1-3}$alkyl ist;

b) Ro ist $C_{1-3}$Alkyl;

R ist

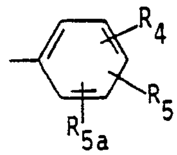

$R_2$ ist Wasserstoff, $C_{1-3}$Alkyl, Methoxy, Fluor oder Chlor oder 4-, 5- oder 6-Benzyloxy;
$R_3$ ist Wasserstoff oder $C_{1-3}$Alkyl;
$R_4$ und $R_5$ sind unabhängig Wasserstoff, Methyl, Methoxy, Fluor oder Chlor;
$R_{5a}$ ist Wasserstoff oder Methyl;
X ist $(CH_2)_2$ oder (E)—CH=CH; und

Z ist 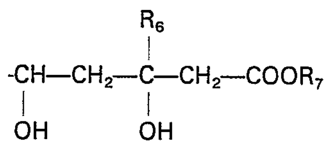

worin
$R_6$ Wasserstoff oder Methyl ist und
$R_7$ Wasserstoff oder $C_{1-2}$Alkyl ist;

c) wie b) mit der Ausnahme, dass $R_6$ Wasserstoff ist;

d) Ro ist $C_{1-3}$Alkyl,
R ist

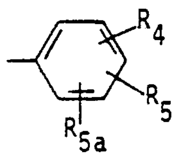

$R_2$ ist Wasserstoff, $C_{1-3}$Alkyl oder 4- oder 6-Benzyloxy;
$R_3$ ist Wasserstoff oder Methyl,
$R_4$ ist Wasserstoff, Methyl oder Fluor,
$R_5$ ist Wasserstoff oder Methyl;
$R_{5a}$ ist Wasserstoff,
X ist (E)—CH=CH und

Z ist 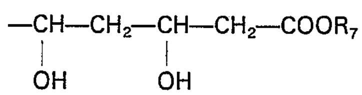

worin
$R_7$ Wasserstoff oder $C_{1-2}$Alkyl ist.

5. Verbindung gemäss Anspruch 4, welche wenn in Salzform in Form des Natrium, Kalium- oder Ammoniumsalzes ist.

6. Verbindung nach einem der Ansprüche 1 bis 5 welche wenn Z in einer anderen als Lakton-Form ist in Erythro-Form ist.

7. Verbindung gemäss Anspruch 1, worin R p-Fluormethyl ist, Ro Isopropyl ist, $R_2$ und $R_3$ Wasserstoff sind, X (E)—CH=CH ist, $R_6$ Wasserstoff ist in Erythro-Form.

8. Verbindung gemäss Anspruch 7 in Form der freien Säure oder in Salz- oder Esterform.

9. Verbindung gemäss Anspruch 8 in Form der freien Säure, des Natriumsalzes oder des Methylesters.

10. Natrium *erythro*-(±)-(E)-3,5-dihydroxy-7-[3'-(4''-fluorphenyl)-1'-(1''-methylethyl)-indol-2'-yl]hept-6-enoat.

11. Natrium *erythro*-(±)-(E)-3,5-dihydroxy-7-[3'-(3'',5''-dimethylphenyl)-1'-(1''-methylethyl)-indol-2'-yl]hept-6-enoat.

12. Verbindung gemäss Anspruch 7 in 3R,5S-Konfiguration und in Form ihres Natriumsalzes.

13. Eine pharmazeutische Zusammensetzung, umfassend eine Verbindung gemäss Anspruch 1 in Form der freien Säure oder in Form eines physiologisch hydrolysierbaren und akzeptablen Esters oder eines Laktones davon oder in Form eines pharmazeutisch akzeptablen Salzes, zusammen mit einem pharmazeutisch akzeptablen Verdünner oder Träger.

14. Eine Verbindung gemäss Anspruch 1, in Form der freien Säure oder in Form eines physiologisch-hydrolysierbaren und akzeptablen Esters oder eines Laktones davon oder in Form eines pharmazeutisch akzeptablen Salzes zur Anwendung als Pharmazeutikum.

15. Eine Verbindung gemäss Anspruch 1, in Form der freien Säure oder in Form eines physiologisch-hydrolysierbaren und akzeptablen Esters oder eines Laktones davon oder in Form eines pharmazeutisch akzeptablen Salzes zur Anwendung bei der Hemmung der Cholesterin-Biosynthese oder zur Behandlung der Atherosklerose.

16. Eine Verfahren zur Herstellung einer Verbindung gemäss Anspruch 1, umfassend

a) Wenn $R_6$ Wasserstoff bedeutet, Reduktion einer Verbindung der Formel V

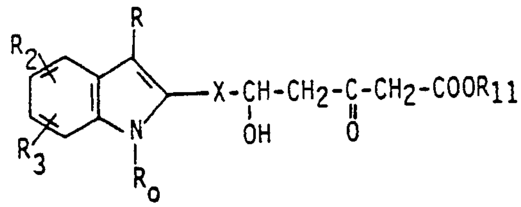

V

worin $R_1$ ein Radikal bedeutet, das einen physiologisch-hydrolysierbaren und akzeptablen ester bildet und X, R, Ro, $R_2$ und $R_3$ wie oben definiert sind,

b) wenn $R_6$ $C_{1-3}$Alkyl bedeutet, Hydrolyse einer Verbindung der Formel XII

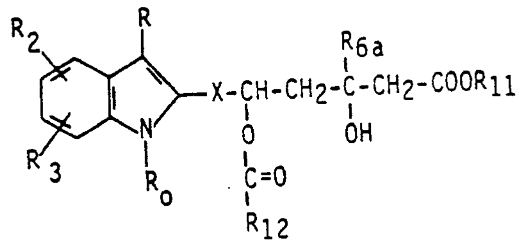

XII

worin $R_{6a}$ $C_{1-3}$Alkyl bedeutet, $R_{12}$ eine esterbildende Gruppe ist und X, R, Ro, $R_3$ und $R_{11}$ wie oben definiert sind,

c) wenn X —CH=CH— ist Entschutzung einer Verbindung der Formel XXVIII

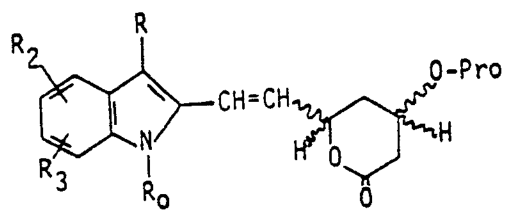

XXVIII

worin Pro eine Schutzgruppe ist und R, Ro, $R_2$ und $R_3$ wie oben definiert sind,

d) Hydrolyse einer Verbindung der Formel I in Form eines physiologisch hydrolisierbaren Esters oder eines Laktones oder

e) Veresterung oder Laktonisierung einer Verbindung der Formel I in Form der freien Säure und falls eine freie Carboxylgruppe anwesend ist, Wiedergewinnung einer so erhaltene Verbindung in Form der freien Säure oder in Form eines Salzes.

17. Verbindung der Formel XX

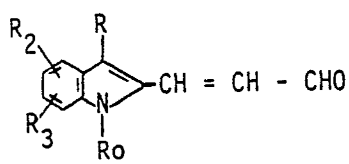

XX

worin R, Ro, $R_2$ und $R_3$ die in einem der Ansprüche 1 bis 5 angegebenen Bedeutungen haben.

**Revendications**

1. Composés de formule I

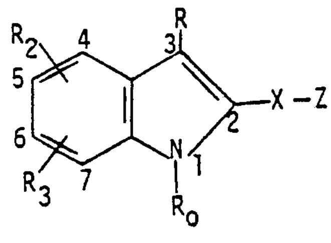

(I)

dans laquelle l'un des symboles R et $R_o$ signifie

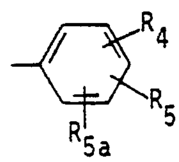

et l'autre signifie un groupe alkyle en $C_1$—$C_6$ primaire ou secondaire, cycloalkyle en $C_3$—$C_6$ ou phényl —$(CH_2)_m$—,
où

$R_4$ signifie l'hydrogène, alkyle en $C_1$—$C_4$, alcoxy en $C_1$—$C_4$ (excepté tert.-butoxy), trifluorométhyle, fluoro, chloro, phénoxy ou benzyloxy,

$R_5$ signifie l'hydrogène, alkyle en $C_1$—$C_3$, alcoxy en $C_1$—$C_3$, trifluorométhyle, fluoro, chloro, phénoxy ou benzyloxy,

$R_{5a}$ signifie l'hydrogène, alkyle en $C_1$—$C_2$, alcoxy en $C_1$—$C_2$, fluoro ou chloro, et

m signifie 1, 2 ou 3, avec des conditions que les deux symboles $R_5$ et $R_{5a}$ signifient l'hydrogène lorsque $R_4$ signifie l'hydrogène, que $R_{5a}$ signifie l'hydrogène lorsque $R_5$ signifie l'hydrogène, qu'au plus l'un des symboles $R_4$ et $R_5$ signifie trifluorométhyle, qu'au plus l'un des symboles $R_4$ et $R_5$ signifie phénoxy et qu'au plus l'un des symboles $R_4$ et $R_5$ signifie benzyloxy,

$R_2$ signifie l'hydrogène, alkyle en $C_1$—$C_4$, cycloalkyle en $C_3$—$C_6$, alcoxy en $C_1$—$C_4$, (excepté tert.-butoxy), trifluorométhyle, fluoro, chloro, phénoxy ou benzyloxy,

$R_3$ signifie l'hydrogène, alkyle en $C_1$—$C_3$, alcoxy en $C_1$—$C_3$, trifluorométhyle, fluoro, chloro, phénoxy ou benzyloxy, avec des conditions que $R_3$ signifie l'hydrogène lorsque $R_2$ signifie l'hydrogène, qu'au plus l'un des symboles $R_2$ et $R_3$ signifie trifluorométhyle, qu'au plus l'un des symboles $R_2$ et $R_3$ signifie phénoxy et qu'au plus l'un des symboles $R_2$ et $R_3$ signifie benzyloxy,

X signifie —$(CH_2)_n$— ou —CH=CH—, (n = 0, 1, 2 ou 3), et

Z signifie

$R_6$
5 4 3 2 1
—CH—$CH_2$—C—$CH_2$—COOH (II)
OH OH

où

$R_6$ sgnifie l'hydrogène ou alkyle en $C_1$—$C_3$,

sous forme d'acide libre ou sous forme d'un ester physiologiquement hydrolysable et physiologiquement acceptable, ou d'une δ-lactone de ce composé, ou sous forme de sel.

2. Un composé selon la revendication 1, dans lequel l'un des symboles R et $R_o$ signifie

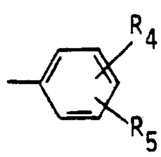

et l'autre signifie un groupe alkyle en $C_1$—$C_3$, n-butyle ou i-butyle,
où

$R_4$ signifie l'hydrogène, alkyle en $C_1$—$C_3$, n-butyle, i-butyle, alcoxy en $C_1$—$C_3$, n-butoxy, i-butoxy, trifluorométhyle, fluoro, chloro, phénoxy ou benzyloxy, et

$R_5$ signifie l'hydrogène, alkyle en $C_1$—$C_3$, alcoxy en $C_1$—$C_3$, trifluorométhyl, fluoro, chloro, phénoxy ou benzyloxy, avec les conditions qu'au plus l'un des symboles $R_4$ et $R_5$ signifie trifluorométhyle, au plus l'un des symboles $R_4$ et $R_5$ signifie phénoxy, et qu'au plus l'un des symboles $R_4$ et $R_5$ signifie benzyloxy,

$R_2$ signifie l'hydrogène, alkyle en $C_1$—$C_3$, n-butyle, i-butyle, alcoxy en $C_1$—$C_3$, n-butoxy, i-butoxy, trifluorométhyle, fluoro, chloro, phénoxy ou benzyloxy,

$R_3$ signifie l'hydrogène, alkyle en $C_1$—$C_3$, alcoxy en $C_1$—$C_3$, trifluorométhyle, fluoro, chloro, phénoxy ou benzyloxy, avec les conditions qu'au plus l'un des symboles $R_2$ et $R_3$ signifie trifluorométhyle, qu'au plus l'un des symboles $R_2$ et $R_3$ signifie phénoxy et qu'au plus l'un des symboles $R_2$ et $R_3$ signifie benzyloxy,

X signifie

$-(CH_2)_n-$ ou 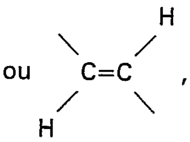 ,

où n signifie 0, 1, 2 ou 3, et

Z signifie

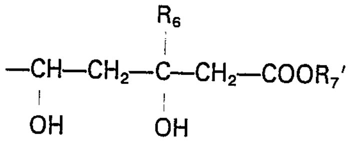 ou 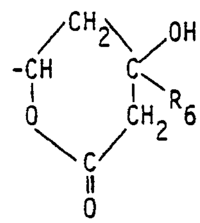

où

$R_6$ signifie l'hydrogène ou alkyle en $C_1$—$C_3$, et

$R_7'$ signifie l'hydrogène, alkyle en $C_1$—$C_3$, n-butyle, i-butyle, t-butyle, benzyle ou M, où M signifie un cation pharmaceutiquement acceptable.

3. Un composé selon la revendication 1 sous forme d'acide libre, de sel ou d'ester.

4. Un composé selon la revendication 1, dans lequel les significations des substituants sont comme suit:

a) Ro signifie un groupe alkyle en $C_1$—$C_6$ primaire ou secondaire ne contenant pas d'atome de carbone asymétrique,

R signifie

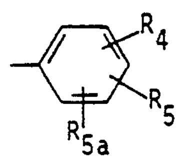

$R_2$ signifie l'hydrogène, alkyle, en $C_1$—$C_3$, alcoxy en $C_1$—$C_3$, trifluorométhyle, fluoro, chloro, phénoxy ou benzyloxy,

$R_3$ signifie l'hydrogène, alkyle en $C_1$—$C_3$, alcoxy en $C_1$—$C_2$, fluoro ou chloro,

$R_4$ signifie l'hydrogène, alkyle en $C_1$—$C_3$, alcoxy en $C_1$—$C_3$, trifluorométhyle, fluoro, chloro, phénoxy ou benzyloxy,

$R_5$ signifie l'hydrogène, alkyle en $C_1$—$C_2$, alcoxy en $C_1$—$C_2$, fluoro ou chloro,

$R_{5a}$ signifie l'hydrogène ou un groupe méthyle,

X signifie $(CH_2)_m$, (où m signifie 1, 2 ou 3) ou (E)—CH=CH, et

Z signifie

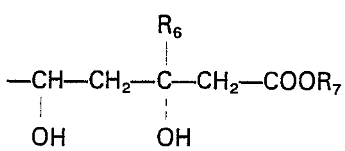

où

$R_6$ signifie l'hydrogène ou alkyle en $C_1$—$C_2$ et

$R_7$ signifie l'hydrogène ou alkyle en $C_1$—$C_3$,

b) Ro signifie alkyle en $C_1$—$C_3$,

R signifie

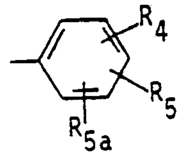

$R_2$ signifie l'hydrogène, alkyle en $C_1$—$C_3$, méthoxy, fluoro ou chloro ou 4-, 5- ou 6-benzyloxy,
$R_3$ signifie l'hydrogène ou alkyle en $C_1$—$C_3$,
$R_4$ et $R_5$ signifient indépendamment l'hydrogène, méthyle, méthoxy, fluoro ou chloro,
$R_{5a}$ signifie l'hydrogène ou méthyle,
X signifie $(CH_2)_2$ ou (E)—CH=CH, et
X signifie

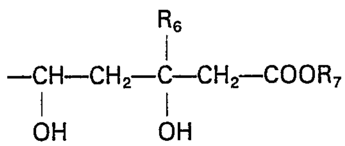

où
$R_6$ signifie l'hydrogène ou méthyle et
$R_7$ signifie l'hydrogène ou alkyle en $C_1$—$C_2$,
c) comme b) excepté que $R_6$ signifie l'hydrogène,
d) Ro signifie alkyle en $C_1$—$C_3$,
R signifie

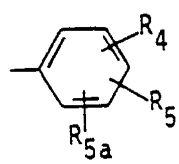

$R_2$ signifie l'hydrogène, alkyle en $C_1$—$C_3$ ou 4- ou 6- benzyloxy,
$R_3$ signifie l'hydrogène ou méthyle,
$R_4$ signifie l'hydrogène, méthyle ou fluoro,
$R_5$ signifie l'hydrogène ou méthyle,
$R_{5a}$ signifie l'hydrogéne et X signifie (E)—CH=CH,
Z signifie

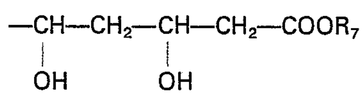

où
$R_7$ signifie l'hydrogène ou alkyle en $C_1$—$C_2$.

5. Un composé selon la revendication 4 qui, lorsqu'il est sous forme de sel, se présente sous forme de sel de sodium, de potassium ou d'ammonium.

6. Un composé selon l'une quelconque des revendciations 1 à 5 qui, lorsque Z est autre que sous forme de lactone, se présente sous forme érythro.

7. Un composé selon la revendication 1, dans lequel R signifie p-fluorophényle, $R_o$ signifie isopropyle, $R_2$ et $R_3$ signifient l'hydrogène, X signifie (E)—CH=CH, $R_6$ signifie l'hydrogène, sous forme érythro.

8. Un composé selon la revendication 7 sous forme d'acide libre, de sel ou d'ester.

9. Un composé selon la revendication 8 sous forme d'acide libre, de sel de sodium ou d'ester méthylique.

10. Erythro - (±) - (E) - 3,5 - dihydroxy - 7 - [3' - (4'' - fluorophényl) - 1' - (1'' - méthyléthyl) - indole - 2' - yl] - hept - 6 - énoate de sodium.

11. Erythro - (±) - (E) - 3,5 - dihydroxy - 7 - [3' - (3'',5'' - diméthylphényl) - 1' - (1'' - méthyléthyl) - indole - 2' - yl] - hept - 6 - énoate de sodium.

12. Un composé selon la revendication 7 de configuration 3R,5S et sous forme de sel de sodium.

13. Une composition pharmaceutique comprenant un composé selon la revendication 1, sous forme d'acide libre ou sous forme d'un ester physiologiquement hydrolysable et physiologiquement acceptable, ou d'une lactone de ce composé, ou sous forme d'un sel pharmaceutiquement acceptable, en association avec un diluant ou véhicule pharmaceutiquement acceptable.

14. Un composé selon la revendication 1, sous forme d'acide libre ou sous forme d'un ester physiologiquement hydrolysable et physiologiquement acceptable, ou d'une lactone de ce composé, ou sous forme d'un sel pharmaceutiquement acceptable, pour l'utilisation comme médicament.

15. Un composé selon la revendication 1, sous forme d'acide libre ou sous forme d'un ester physiologiquement hydrolysable et physiologiquement acceptable, ou d'une lactone de ce composé, ou sous forme d'un sel pharmaceutiquement acceptable, pour l'utilisation dans l'inhibition de la biosynthèse du cholestérol ou pour le traitement de l'athérosclérose.

16. Un procédé de préparation d'un composé selon la revendication 1, qui comprend

a) lorsque $R_6$ signifie l'hydrogène, la réduction d'un composé de formule V

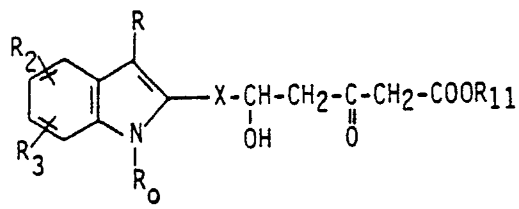

V

dans laquelle $R_{11}$ signifie un radical formant un ester physiologiquement hydrolysable et physiologiquement acceptable et X, R, Ro, $R_2$ et $R_3$ sont tels que définis ci-dessus,

b) lorsque $R_6$ = alkyle en $C_1$—$C_3$, l'hydrolyse d'un composé de formule XII

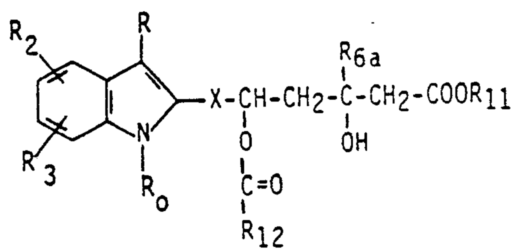

XII

dans laquelle $R_{6a}$ signifie alkyle en $C_1$—$C_3$, $R_{12}$ signifie un groupe formant ester et X, R, Ro, $R_3$ et $R_{11}$ sont tels que définis ci-dessus,

c) lorsque X signifie —CH=CH—, la dèprotection d'un composé de formule XXVIII

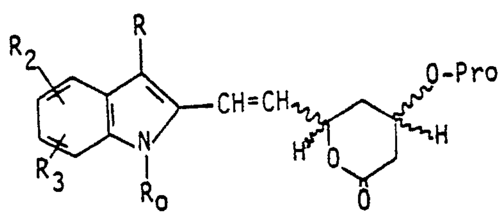

XXVIII

dans laquelle Pro signifie un groupe protecteur et R, Ro, $R_2$ et $R_3$ sont tels que dèfinis ci-dessus,

d) l'hydrolyse d'un composé de formule I sous forme d'un ester physiologiquement hydrolysable ou d'une lactone, ou

e) l'estérification ou la lactonisation d'un composé de formule I sous forme d'acide libre,

et lorsque un groupe carboxy libre est présent, la récupération du composé obtenu sous forme libre ou sous forme d'un sel.

17. Un composé de formule XX

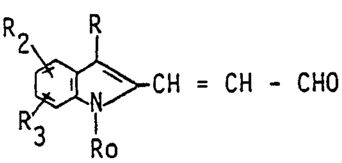

XX

dans laquelle R, Ro, $R_2$ et $R_3$ ont les significations données à l'une quelconque des revendications 1 à 5.